# EUROPEAN PATENT SPECIFICATION

(11) **EP 4 253 559 B1**
(45) Date of publication and mention of the grant of the patent: **02.04.2025**
(21) Application number: 23180094.7
(22) Date of filing: 24.02.2021
(51) Int. Cl.: C12Q 1/6806

(54) **KITS FOR GENOTYPING**
KITS ZUR GENOTYPISIERUNG
KITS POUR LE GÉNOTYPAGE

(30) Priority: 26.02.2020 US 202062981866 P
(43) Date of publication of application: 04.10.2023
(62) Divisional of application: 21713210.9
(73) Proprietor: Illumina, Inc., San Diego, CA 92122 (US); Illumina Cambridge Limited, Cambridge CB21 6DF (GB)
(72) Inventor: SLATTER, Andrew, Cambridge, CB21 6DF (GB); VERMAAS, Eric Hans, San Diego, CA 92122 (US)
(74) Representative: Forresters IP LLP

(56) References cited:
- WO-A1-2016/075204
- WO-A1-2016/162309
- WO-A1-2018/161019
- ERIK S. HOPMANS ET AL: "A programmable method for massively parallel targeted sequencing", NUCLEIC ACIDS RESEARCH, vol. 42, no. 10, 29 April 2014 (2014-04-29), pages e88 - e88, XP055544838, ISSN: 0305-1048, DOI: 10.1093/nar/gku282
- SAMUEL MYLLYKANGAS ET AL: "Efficient targeted resequencing of human germline and cancer genomes by oligonucleotide-selective sequencing", NATURE BIOTECHNOLOGY, vol. 29, no. 11, 23 October 2011 (2011-10-23), New York, pages 1024 - 1027, XP055544835, ISSN: 1087-0156, DOI: 10.1038/nbt.1996

## Description

### BACKGROUND

The detection of specific nucleic acids can be used for diagnostic medicine and molecular biology research. Gene probe assays may be useful, for example, in identifying infectious organisms, such as bacteria and viruses; in probing the expression of normal and mutant genes and identifying mutant genes, such as oncogenes; in typing tissue for compatibility preceding tissue transplantation; in matching tissue or blood samples for forensic medicine; and for exploring homology among genes from different species.

Hopmans et al., Nucleic Acids Res, 2014 Jun;42(10): e88, discloses oligonucleotide-selective sequencing approaches, wherein the surface bound primers are extended using target specific oligonucleotide templates, to provide target specific capture, and wherein the captured nucleic acids are amplified on a flow cell and then sequenced. WO 2018/161019 A1 discloses a method for selecting a critical parameter for direct targeted sequencing including hybridizing probes in a capture probe library to surface-bound oligonucleotides. WO 2016/075204 A1 discloses methods for capturing and amplifying target polynucleotides on a solid surface, in particular in a well in a microarray, as well as methods for modifying immobilized capture primers.

### SUMMARY

The present invention relates to a genotyping probe fluid, a kit, and a method according to the appended claims.

Disclosed herein are genotyping oligonucleotides that include specific nucleotide sequence sections designed to have a designated function in cluster formation, linearization, target locus identification, and/or sequencing. The genotyping oligonucleotides enable genotyping to take place on non-patterned or patterned flow cells, and do not involve extra immobilization components.

A first aspect disclosed herein is a kit comprising: a flow cell, including: a substrate; and first and second capture primers attached to the substrate; and a genotyping probe fluid, including: a liquid carrier; and a genotyping oligonucleotide in the liquid carrier, the genotyping oligonucleotide including: a first primer sequence; a probe sequence representative of a target genotyping locus; a restriction endonuclease site; and a second primer sequence that is at least partially complementary to the second capture primer.

It is to be understood that any features of the kit disclosed herein may be combined together in any desirable manner and/or configuration to achieve the benefits as described in this disclosure, including, for example, to achieve genotyping on a flow cell.

A second aspect disclosed herein is a method comprising: introducing a genotyping probe fluid to a flow cell including first and second capture primers, the genotyping probe fluid including a plurality of genotyping oligonucleotides, each of the genotyping oligonucleotides including: a first primer sequence; a probe sequence representative of a respective target genotyping locus; a restriction endonuclease site; and a second primer sequence that is at least partially complementary to the second capture primer; whereby a respective genotyping oligonucleotide reacts to produce respective clonal populations of amplicons from the respective genotyping oligonucleotide; linearizing the amplicons to produce probe templates; sequencing at least one probe identifying section of the probe templates to identify each of the probe sequences; removing at least respective nascent strands from the probe templates, whereby a 3' OH group at an end of the probe templates is exposed; hybridizing respective samples to the probe templates; and performing respective genotyping reactions of the samples at the exposed 3' OH groups.

It is to be understood that any features of the method may be combined together in any desirable manner. Moreover, it is to be understood that any combination of features of the method and/or of the kit may be used together, and/or combined with any of the examples disclosed herein to achieve the benefits as described in this disclosure, including, for example, to achieve genotyping on a flow cell.

### BRIEF DESCRIPTION OF THE DRAWINGS

Features of examples of the present disclosure will become apparent by reference to the following detailed description and drawings, in which like reference numerals correspond to similar, though perhaps not identical, components. For the sake of brevity, reference numerals or features having a previously described function may or may not be described in connection with other drawings in which they appear.
Fig. 1A is a schematic illustration of an example of the genotyping oligonucleotide disclosed herein;
Fig. 1B is a schematic illustration of another example of the genotyping oligonucleotide disclosed herein;
Fig. 2A is a top view of an example of a flow cell;
Fig. 2B is an enlarged, and partially cutaway view of an example of a flow channel of the flow cell including depressions formed along the flow channel;
Figs. 3A through 3H schematically depict an example of a method disclosed herein; and
Figs. 4A through 4H schematically depict another example of a method disclosed herein.

### DETAILED DESCRIPTION

Disclosed herein are genotyping oligonucleotides that include specific nucleotide sequence sections. Each nucleotide sequence section of the genotyping oligonucleotide is designed to have a designated function in cluster formation, linearization, target locus identification, and/or sequencing.

The genotyping oligonucleotides can be used in a non-patterned flow cell having capture primers on a surface thereof, or in a patterned flow cell including depressions having capture primers therein. At different areas on the non-patterned flow cell surface or within respective depressions of the patterned flow cell, monoclonal populations (clusters) of amplicons can be generated from respective genotyping oligonucleotides. The amplicons in a particular area or depression may be different from the amplicons in each of the other areas or depressions, and thus each monoclonal population of amplicons can represent a unique target locus for genotyping.

The genotyping oligonucleotides disclosed herein enable genotyping to take place on non-patterned flow cells or on patterned flow cells, and do not involve extra immobilization components, such as solid phase beads. Additionally, the genotyping oligonucleotides disclosed herein may be suitable for use with any flow cell surface that utilizes clonally amplified clusters.

Also disclosed herein are methods for preparing a target genotyping locus, which may be used with the genotyping oligonucleotides. These methods are amplification methods that generate genomic DNA (gDNA) fragments without having to introduce a cleavage site and perform fragmentation.

### Definitions

Terms used herein will be understood to take on their ordinary meaning in the relevant art unless specified otherwise. Several terms used herein and their meanings are set forth below.

As used herein, the singular forms "a," "an," and "the" refer to both the singular as well as plural, unless the context clearly indicates otherwise. The term "comprising" as used herein is synonymous with "including," "containing," or "characterized by," and is inclusive or open-ended and does not exclude additional, unrecited elements or method steps.

Reference throughout the specification to "one example," "another example," "an example," and so forth, means that a particular element (e.g., feature, structure, composition, configuration, and/or characteristic) described in connection with the example is included in at least one example described herein, and may or may not be present in other examples. In addition, it is to be understood that the described elements for any example may be combined in any suitable manner in the various examples unless the context clearly dictates otherwise.

The terms "substantially" and "about" used throughout this disclosure, including the claims, are used to describe and account for small fluctuations, such as due to variations in processing. For example, these terms can refer to less than or equal to ±10% from a stated value, such as less than or equal to ±5% from a stated value, such as less than or equal to ±2% from a stated value, such as less than or equal to ±1% from a stated value, such as less than or equal to ±0.5% from a stated value, such as less than or equal to ±0.2% from a stated value, such as less than or equal to ±0.1% from a stated value, such as less than or equal to ±0.05% from a stated value.

Furthermore, it is to be understood that the ranges provided herein include the stated range and any value or sub-range within the stated range, as if they were explicitly recited. For example, a range represented by from about 2 mm to about 300 mm, should be interpreted to include not only the explicitly recited limits of from about 2 mm to about 300 mm, but also to include individual values, such as about 15 mm, 22.5 mm, 245 mm, etc., and sub-ranges, such as from about 20 mm to about 225 mm, etc.

*Attached:* The state of two things being joined, fastened, adhered, connected or bound to each other, either directly or indirectly. For example, a nucleic acid can be attached to a functionalized polymer by a covalent or non-covalent bond. A covalent bond is characterized by the sharing of pairs of electrons between atoms. A non-covalent bond is a physical bond that does not involve the sharing of pairs of electrons and can include, for example, hydrogen bonds, ionic bonds, van der Waals forces, hydrophilic interactions and hydrophobic interactions. When two things are directly attached, there is not an intervening component. For example, the first capture primer and the probe sequence in some examples of the genotyping oligonucleotide are directly covalently bound to each other. When two things are indirectly attached, there is some intervening component. For example, the first capture primer and the probe sequence in some examples of the genotyping oligonucleotide are indirectly bound to each other through an index sequence portion and a priming site portion.

*Depositing:* Any suitable application technique, which may be manual or automated, and, in some instances, results in modification of the surface properties. Generally, depositing may be performed using vapor deposition techniques, coating techniques, grafting techniques, or the like. Some specific examples include chemical vapor deposition (CVD), spray coating (e.g., ultrasonic spray coating), spin coating, dunk or dip coating, doctor blade coating, puddle dispensing, flow through coating, aerosol printing, screen printing, microcontact printing, inkjet printing, or the like.

*Depression:* A discrete concave feature in a substrate or a patterned resin having a surface opening that is at least partially surrounded by interstitial region(s) of the substrate or the patterned resin. Depressions can have any of a variety of shapes at their opening in a surface including, as examples, round, elliptical, square, polygonal, star shaped (with any number of vertices), etc. The cross-section of a depression taken orthogonally with the surface can be curved, square, polygonal, hyperbolic, conical, angular, etc. As examples, the depression can be a well or two interconnected wells. The depression may also have more complex architectures, such as ridges, step features, etc.

*Each:* When used in reference to a collection of items, each identifies an individual item in the collection, but does not necessarily refer to every item in the collection. Exceptions can occur if explicit disclosure or context clearly dictates otherwise.

*Flow Cell:* A vessel having a chamber (e.g., a flow channel) where a reaction can be carried out, an inlet for delivering reagent(s) to the chamber, and an outlet for removing reagent(s) from the chamber. In some examples, the chamber enables the detection of the reaction that occurs in the chamber. For example, the chamber can include one or more transparent surfaces allowing for the optical detection of arrays, optically labeled molecules, or the like.

*Genomic DNA (gDNA):* One or more chromosomal polymeric deoxyribonucleotide molecules occurring naturally in the nucleus of a eukaryotic cell or in a prokaryote, virus, mitochondrion or chloroplast and containing sequences that are naturally transcribed into RNA as well as sequences that are not naturally transcribed into RNA by the cell. A gDNA of a eukaryotic cell contains at least one centromere, two telomeres, one origin of replication, and one sequence that is not transcribed into RNA by the eukaryotic cell including, for example, an intron or transcription promoter. A gDNA of a prokaryotic cell contains at least one origin of replication and one sequence that is not transcribed into RNA by the prokaryotic cell including, for example, a transcription promoter. A eukaryotic genomic DNA can be distinguished from prokaryotic, viral or organellar genomic DNA, for example, according to the presence of introns in eukaryotic genomic DNA and absence of introns in the gDNA of the others.

*Genotyping oligonucleotide:* A single stranded deoxyribonucleic acid sequence that includes specific nucleotide sequence sections, one of which is a probe sequence representing a target locus for genotyping. The genotyping oligonucleotide can serve as a template for cluster generation.

*Index Sequence Portion:* A short strand, ranging from 10 to 30 nucleobases, that identifies the probe sequence in some examples of the genotyping oligonucleotide.

*Locus or Loci:* A sequence-specific location in a nucleic acid sample. The term can include predetermined or predicted nucleic acid sequences expected to be present in isolated nucleic acid molecules. These predetermined or predicted nucleic acid sequences may be referred to herein as a "target genotyping locus," and they may be region(s) of interest for analysis. The term is meant to encompass single nucleotide polymorphisms (SNPs), mutations, variable number of tandem repeats (VNTRs) and single tandem repeats (STRs), other polymorphisms, insertions, deletions, splice variants or any other known genetic markers.

*Nucleic acid*: An oligomeric or polymeric form of nucleotides of any length, and may include deoxyribonucleotides, analogs thereof, or mixtures thereof. The term may refer to single stranded or double stranded oligonucleotides or polynucleotides.

*Nucleotide:* A nitrogen containing heterocyclic base (a nucleobase), a sugar, and one or more phosphate groups. Nucleotides are monomeric units of a nucleic acid sequence. In ribonucleotides (RNA), the sugar is a ribose, and in deoxyribonucleotides (DNA), the sugar is a deoxyribose, i.e., a sugar lacking a hydroxyl group that is present at the 2' position in ribose. The nitrogen containing heterocyclic base (i.e., nucleobase) can be a purine base or a pyrimidine base. Purine bases include adenine (A) and guanine (G), and modified derivatives or analogs thereof. Pyrimidine bases include cytosine (C), thymine (T), and uracil (U), and modified derivatives or analogs thereof. The C-1 atom of deoxyribose is bonded to N-1 of a pyrimidine or N-9 of a purine. Naturally occurring nucleotides generally have a backbone containing phosphodiester bonds. A nucleic acid analog may have any of the phosphate backbone, the sugar, or the nucleobase altered. Examples of nucleic acid analogs include, for example, universal bases or phosphate-sugar backbone analogs, such as peptide nucleic acid (PNA).

*Nucleotide sequence section*: A portion of the genotyping oligonucleotide. Each portion of the genotyping oligonucleotide may be designed so that it is involved in specific process(es) in the method(s) disclosed herein.

*Primer*: A single stranded deoxyribonucleic acid sequence that can hybridize to a specific sequence. One example of a primer is a "capture primer." A capture primer may be present in depressions of a flow cell and can hybridize to a primer sequence of a genotyping oligonucleotide or an amplicon thereof. The capture primer can serve as a starting point for amplification and cluster generation. Another example of a primer is a "sequencing primer." A sequencing primer can hybridize to a portion of a single stranded probe template in order to prime synthesis of at least a portion of the single stranded probe template. Other primers, such as random primers, may be used in a random primer amplification reaction for generating gDNA fragments, such as the target genotyping locus. Any primer can include any combination of nucleotides or analogs thereof. The primer length can be any number of bases long and can include a variety of non-natural nucleotides. In an example, the capture primer or the sequencing primer is a short strand, ranging from 10 to 60 nucleobases, or from 20 to 40 nucleobases.

*Priming Sequence Portion:* A priming site for sequencing of an index sequence portion, both of which are included in some examples of the genotyping oligonucleotide.

*Probe Sequence:* A specific section of the genotyping oligonucleotide that includes a nucleotide sequence which represents a target locus for genotyping. The probe sequence, or an amplicon thereof, includes from 25 to 50 nucleobases having a specific order that is complementary to, and thus can hybridize to the target locus sequence.

*Single Stranded Probe Template:* A single stranded deoxyribonucleic acid sequence that is generated during clustering. The genotyping oligonucleotide serves as a template for cluster generation, and thus the single stranded probe templates are amplicons, or portions of amplicons of the genotyping oligonucleotide.

### Genotyping Oligonucleotides

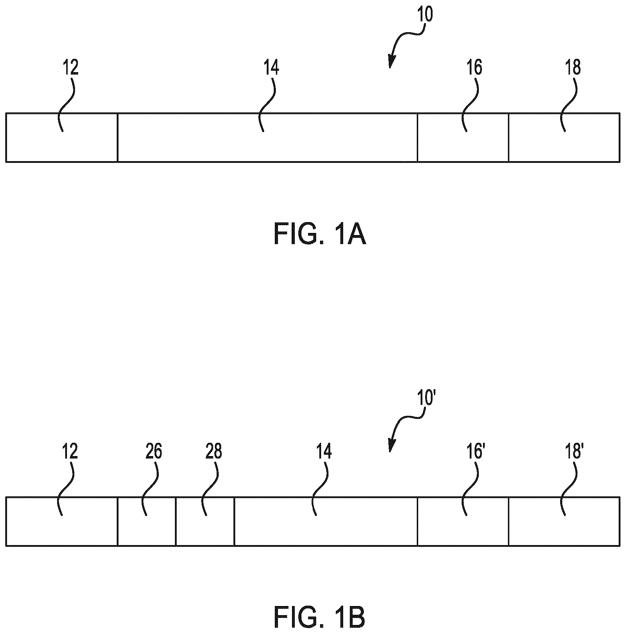

Fig. 1A and Fig. 1B schematically depict two different examples of the genotyping oligonucleotides 10, 10'. Each of the genotyping oligonucleotides 10, 10' includes specific nucleotide sequence sections, which include a first primer sequence 12, a probe sequence 14, a restriction endonuclease site 16 or 16', and a second primer sequence 18 or 18'.

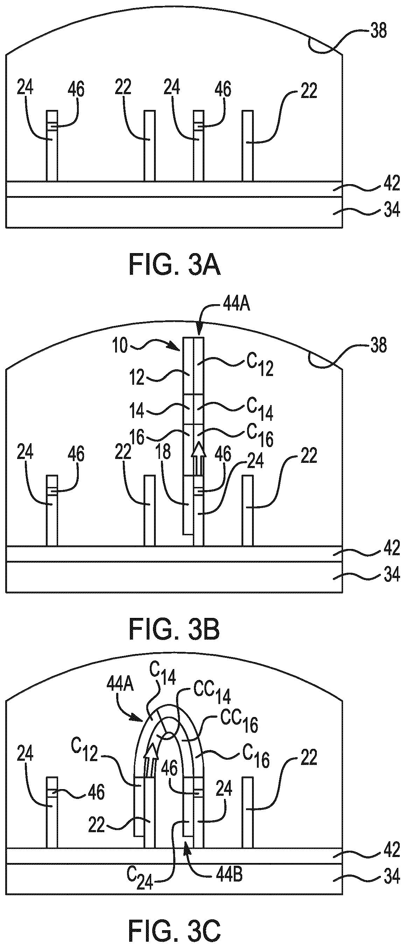

The example genotyping oligonucleotide 10 shown in Fig. 1A includes the first primer sequence 12 directly and covalently attached to the probe sequence 14, which is directly and covalently attached to the restriction endonuclease site 16, which is directly and covalently attached to the second primer sequence 18. An example of the method involving these genotyping oligonucleotides 10 is shown and described in reference to Fig. 3A through Fig. 3H.

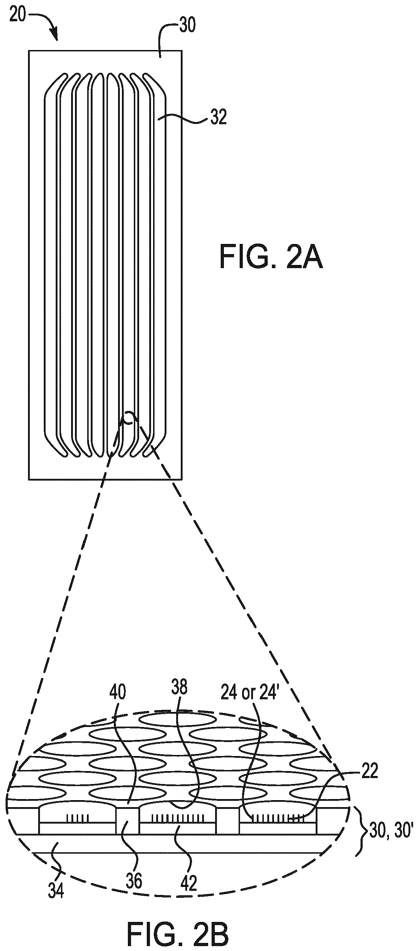

The first primer sequence 12 of the genotyping oligonucleotide 10 may have the same polarity and the same sequence as a first capture primer 22 (see Fig. 2B) that is present on the surface of a flow cell 20 (see Fig. 2A and Fig. 2B). As such, the number, order, and type of nucleobases in at least a portion of the first primer sequence 12 depends upon the number, order, and type of nucleobases in the first capture primer 22. During clustering, amplicons of the genotyping oligonucleotides 10 are generated that include a sequence complementary to the first primer sequence 12 (e.g., P5'). This complementary portion (shown as C₁₂ in Fig. 3B) can hybridize to the first capture primer 22.

In an example, the first capture primer 22 has a universal sequence for capture and/or amplification purposes. An example of the first capture primer 22 includes P5 primers, examples of which are used on the surface of commercial flow cells sold by Illumina Inc. for sequencing, for example, on HISEQ^{™}, HISEQX^{™}, MISEQ^{™}, MISEQDX^{™}, MINISEQ^{™}, NEXTSEQ^{™}, NEXTSEQDX^{™}, NOVASEQ^{™}, ISEQ^{™}, GENOME ANALYZER^{™}, and other instrument platforms. In another example, the first capture primer 22 includes the following:
First capture primer: 5' → 3'
AATGATACGGCGACCACCGA (SEQ. ID. NO. 1)

The first primer sequence 12 may have the same sequence as the first capture primer 22. While an example has been provided, it is to be understood that other sequences may be used for the first primer sequence 12 and for the first capture primer 22. The first primer sequence 12 may also be at least partially the same as the first capture primer 22. By "at least partially the same," it is meant that a sufficient number of nucleobases in the first primer sequence 12 and in the first capture primer 22 are the same so that hybridization can occur between the two 12, 22. The first primer sequence 12 of the genotyping oligonucleotide 10 is directly attached to the probe sequence 14. The probe sequence 14 is representative of a target genotyping locus. Therefore, the probe sequence 14 is capable of hybridizing to the target locus sequence during genotyping.

The restriction endonuclease site 16 of the genotyping oligonucleotide 10 is directly attached to the probe sequence 14. This restriction endonuclease site 16 provides the genotyping oligonucleotide 10 with a digestion site. In an example, the restriction endonuclease site 16 is sensitive to a restriction enzyme selected from the group consisting of a 4 base cutter restriction endonuclease, a 5 base cutter restriction endonuclease, a 6 base cutter restriction endonuclease. Some example 4 base cutters include DpnII, FatI, MluCl, BfuCl, Mbol, Sau3Al, Bfal, BstUI, PmII, Kasl, and others that are commercially available, for example, from New England BioLabs Inc., ThermoFisher Scientific, etc. Some example 5 base cutters include BssKI, StyD41, MaeIII, PspGI, Ddel, Fmul, PspGI, Tfil, and others that are commercially available, for example, from New England BioLabs Inc., ThermoFisher Scientific, etc. Some example 6 base cutters include AcII, Afel, Nspl, Haell, TatI, and others that are commercially available, for example, from New England BioLabs Inc., ThermoFisher Scientific, etc. The restriction endonuclease site 16 is positioned such that after cleavage/digestion with a restriction enzyme, the last base of the probe sequence 14 is the final 3' OH that is exposed for the sequencing/genotyping reaction.

The second primer sequence 18 of the genotyping oligonucleotide 10 is at least partially complementary to the second capture primer 24 (see Fig. 2B) that is present on the surface of a flow cell 20. By "at least partially complementary," it is meant that a sufficient number of nucleobases in the second primer sequence 18 and in the second capture primer 24 are complementary so that hybridization can occur between the two 18, 24. As such, the number, order, and type of nucleobases in the second primer sequence 18 depend upon the number, order, and type of nucleobases in the second capture primer 24.

In an example, the second capture primer 24 has a universal sequence for capture and/or amplification purposes. An example of the second capture primer 24 includes P7 primers, examples of which are used on the surface of commercial flow cells sold by Illumina Inc. for sequencing, for example, on HISEQ^{™}, HISEQX^{™}, MISEQ^{™}, MISEQDX^{™}, MINISEQ^{™}, NEXTSEQ^{™}, NEXTSEQDX^{™}, NOVASEQ^{™}, ISEQ^{™} GENOME ANALYZER^{™}, and other instrument platforms. In another example, the second capture primer 24 includes the following:
Second capture primer: 5' → 3'
CAAGCAGAAGACGGCATACGA (SEQ. ID. NO. 2)

The second primer sequence 18 is at least partially complementary to the sequence of the second capture primer 24. In this example, the second primer sequence 18 may include the following:
Second primer sequence: 5' → 3'
GTTCGTCTTCTGCCGTATGCT (SEQ. ID. NO. 3)

While an example has been provided, it is to be understood that other sequences may be used for the second primer sequence 18 and for the second capture primer 24.

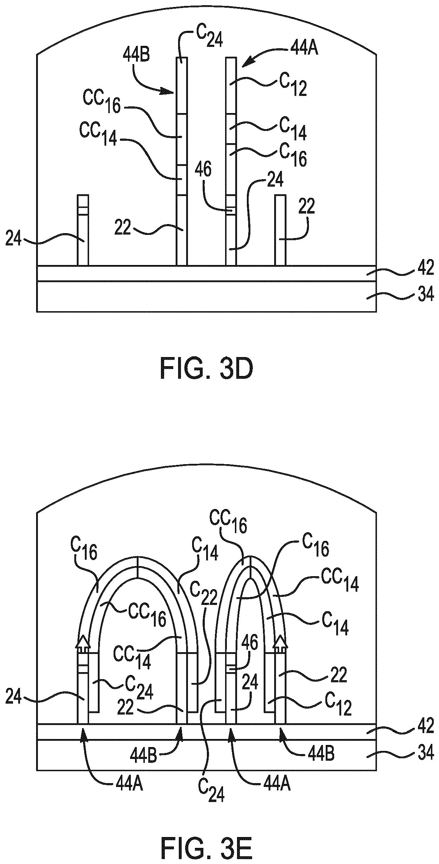

The second capture primer 24 on the flow cell 20 also includes a cleavage site (see reference numeral 46 in Fig. 3A). The cleavage site may be used to linearize bridged probe templates after cluster formation (described more in reference to Fig. 3E). The chemistry of the cleavage site of the second capture primer 24 may be different from the chemistry of the restriction endonuclease site 16 of the genotyping nucleotide 10 so that premature digestion of the restriction endonuclease site 16 does not take place. The cleavage site and restriction endonuclease site 16 have separate and specific cleavage reactions. These reactions may be considered orthogonal, in that one reaction does not initiate, affect, or otherwise interfere with the other reaction. Examples of suitable cleavage sites for the second capture primer 24 include enzymatically cleavable nucleobases or chemically cleavable nucleobases, modified nucleobases, or linkers (e.g., attached between nucleobases). The enzymatically cleavable nucleobase may be susceptible to cleavage by reaction with a glycosylase and an endonuclease, or with an exonuclease. One specific example of the cleavable nucleobase is deoxyuracil (dU), which can be targeted by the USER enzyme. In an example, the uracil base may be incorporated at the 7^{th} base position from the 3' end of the second capture primer 24. Other abasic sites may also be used. Examples of the chemically cleavable nucleobases, modified nucleobases, or linkers include 8-oxoguanine, a vicinal diol, a disulfide, a silane, an azobenzene, a photocleavable group, allyl T (a thymine nucleotide analog having an allyl functionality), allyl ethers, or an azido functional ether.

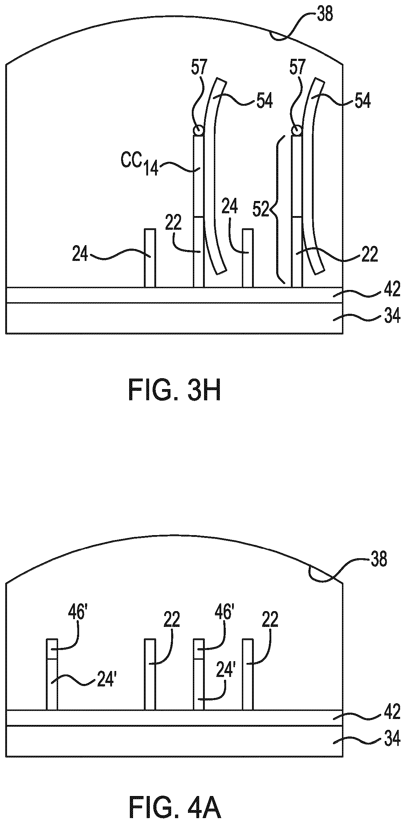

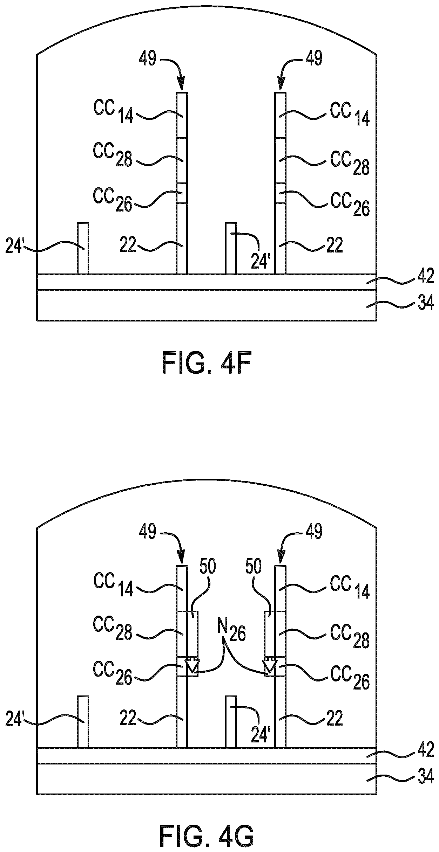

Referring now to Fig. 1B, another example of the genotyping oligonucleotide 10' is schematically depicted. The example genotyping oligonucleotide 10' shown in Fig. 1B includes more nucleotide sequence sections than the genotyping oligonucleotide 10 shown in Fig. 1A. Specifically, the genotyping oligonucleotide 10' further comprises an index sequence portion 26 and a priming site portion 28. In this example, the genotyping oligonucleotide 10' includes the first primer sequence 12 directly and covalently attached to the index sequence portion 26, which is directly and covalently attached to the priming site portion 28, which is directly and covalently attached to the probe sequence 14, which is attached to the second primer sequence 18'. In this example, the restriction endonuclease site 16' may be positioned between the probe sequence 14 and the second primer sequence 18', or may be integrated into the second primer sequence 18'. An example of the method involving these genotyping oligonucleotides 10' is shown and described in reference to Fig. 4A through Fig. 4H.

The first primer sequence 12 of the genotyping oligonucleotide 10' may be any example set forth herein for the genotyping oligonucleotide 10.

The first primer sequence 12 of the genotyping oligonucleotide 10' is directly attached to the index sequence portion 26. The index sequence portion 26 is unique to the probe sequence 14 in the genotyping oligonucleotide 10'. As such, the index sequence portion 26 provides an identifier or distinct barcode that can be used to identify the target locus sequence represented by the probe sequence 14.

The index sequence portion 26 is directly attached to the priming site portion 28. The priming site portion 28 can hybridize to a sequencing primer that initiates nucleotide introduction along the index sequence portion 26 in a template dependent fashion one nucleobase at time.

The priming site portion 28 of the genotyping oligonucleotide 10' is directly attached to the probe sequence 14. As described herein, the probe sequence 14 is capable of hybridizing to the target locus sequence during genotyping.

In the genotyping oligonucleotide 10', the probe sequence 14 is attached to the second primer sequence 18'. In some examples, the probe sequence 14 is indirectly attached to the second primer sequence 18', and the restriction endonuclease site 16' is positioned between the two sections 14, 18'. In other examples, the probe sequence 14 is directly attached to the second primer sequence 18', and the restriction endonuclease site 16' is integrated into the second primer sequence 18'. In either of these examples, the restriction endonuclease site 16' may be sensitive to a Type IIS restriction enzyme. The Type IIS restriction enzyme may be methyl sensitive or may not be methyl sensitive. Type IIS restriction enzymes are a specific group of enzymes that recognize asymmetric DNA sequences (e.g., the restriction endonuclease site 16') and cleave at a defined distance (e.g., from 1 nucleotide to about 20 nucleotides) outside of the recognition sequence. Some examples of Type IIS restriction enzymes that are not methyl sensitive are selected from the group consisting of BbvI (BseXI), Bmrl, Bvel (BspMI), etc. Some Type IIS restriction enzymes that are methyl sensitive are selected from the group consisting of SfaNI, FoKI, HGAI, etc. While some examples have been provided, any suitable Type IIS restriction enzyme may be used, depending upon the recognition sequence of the restriction endonuclease site 16'. Methyl and non-methyl sensitive enzymes are commercially available, for example, from New England BioLabs Inc., ThermoFisher Scientific, etc.

When the restriction endonuclease site 16' is attached at the end of the second primer sequence 18', any example of the second primer sequence 18 may be used. When the restriction endonuclease site 16' is integrated into the second primer sequence 18', the restriction endonuclease site 16' may be introduced at any desirable position along the length of the second primer sequence 18'. The position may depend upon the position of a second restriction endonuclease site along the second capture primer 24', the defined cleavage distance of the Type IIS restriction enzyme to be used, as well as where cleavage is desired along the genotyping oligonucleotide 10' (or an amplicon thereof). This cleavage takes place prior to a genotyping reaction (during linearization as discussed further herein), and renders the genotyping oligonucleotide 10' or an amplicon thereof ready for analysis of a target genotyping locus at a nucleobase of interest. As an example, during a genotyping reaction, a single stranded DNA sample, e.g., the target genotyping locus is hybridized, e.g., to the amplicon, and a single sequencing-by-synthesis reaction is performed to identify the nucleobase of interest. For this analysis to occur, the amplicon should be cleaved at a defined position, where the next nucleobase to be sequenced is complementary to the nucleobase of interest. As such, the restriction endonuclease site 16' is positioned such that after cleavage/digestion with a restriction enzyme, the last base of the probe sequence 14 is the final 3' OH that is exposed for the sequencing/genotyping reaction. In one example, the restriction endonuclease site 16' may be integrated anywhere from the 7^{th} base position to the 15^{th} base position from the 3' end of the second primer sequence 18'.

With the genotyping oligonucleotide 10', the second primer sequence 18' is complementary to the second capture primer 24' (see Fig. 2B) that is present on the surface of a flow cell 20. As such, the genotyping oligonucleotide 10' can hybridize to the second capture primer 24' on the flow cell 20.

The second capture primer 24' may also include the second restriction endonuclease site (see reference numeral 46' in Fig. 4A), wherein the second restriction endonuclease site is complementary to the restriction endonuclease site 16' of the genotyping oligonucleotide 10'. The asymmetric sequences may be sensitive to the Type IIS restriction endonucleases. Cleavage within some predefined distance of the hybridized restriction endonuclease sites 16', 46' is performed during linearization (as mentioned above and described further in reference to Fig. 4F). This removes strands that are not to be genotyped. The position of the second restriction endonuclease site 46' may be at the end of, or integrated into the second capture primer 24', as long as the two restriction endonuclease sites 16', 46' can hybridize and form the recognition site of the Type IIS restriction endonuclease.

Either example of the genotyping oligonucleotide 10, 10' may be prepared using oligonucleotide synthesis processes.

### Flow Cells

The genotyping oligonucleotides 10, 10' may be used with any patterned flow cell 20. While not shown, it is to be understood that other non-patterned flow cells (e.g., which do not include depressions) that utilize the clustering chemistry disclosed herein may alternatively be used in the examples disclosed herein. With non-patterned flow cells, clusters may be identified using software.

An example of a patterned flow cell 20 is depicted in Fig. 2A, and an example of the patterned architecture within the flow cell 20 is shown in Fig. 2B. The flow cell 20 includes a substrate 30 that at least partially defines a lane or flow channel 32.

The substrate 30 may be a single layer/material. Examples of suitable single layer substrates include epoxy siloxane, glass, modified or functionalized glass, plastics (including acrylics, polystyrene and copolymers of styrene and other materials, polypropylene, polyethylene, polybutylene, polyurethanes, polytetrafluoroethylene (such as TEFLON^{®} from Chemours), cyclic olefins/cyclo-olefin polymers (COP) (such as ZEONOR^{®} from Zeon), polyimides, etc.), nylon (polyamides), ceramics/ceramic oxides, silica, fused silica, or silica-based materials, aluminum silicate, silicon and modified silicon (e.g., boron doped p+ silicon), silicon nitride (Si₃N₄), silicon oxide (SiO₂), tantalum pentoxide (Ta₂O₅) or other tantalum oxide(s) (TaOₓ), hafnium oxide (HfO₂), carbon, metals, inorganic glasses, or the like.

When the substrate 30 is a single layer, the depressions 38 (see Fig. 2B) are defined in the single layer.

As shown in Fig. 2B, the substrate 30 may also be a multi-layered substrate 30'. Some examples of the multi-layered substrate 30' include glass or silicon, with a coating layer of tantalum oxide or another ceramic oxide at the surface. Other examples of the multi-layered substrate 30' may include a silicon-on-insulator (SOI) substrate. In the example shown in Fig. 2B, the multi-layered substrate 30' includes an underlying support 34 (e.g., glass or silicon) and a patterned material 36 positioned on the support 34.

The patterned material 36 defines the depressions 38, which are separated by interstitial regions 40. The depressions 38 are located within each of the flow channel(s) 32.

It is to be understood that any material that can be selectively deposited, or deposited and patterned to form the depressions 38 and the interstitial regions 40 may be used for the patterned material 36.

As one example, an inorganic oxide may be selectively applied to the support 34 via vapor deposition, aerosol printing, or inkjet printing. Examples of suitable inorganic oxides include tantalum oxide (e.g., Ta₂O₅), aluminum oxide (e.g., Al₂O₃), silicon oxide (e.g., SiO₂), hafnium oxide (e.g., HfO₂), etc.

As another example, a resin may be applied to the support 34 and then patterned. Suitable deposition techniques include chemical vapor deposition, dip coating, dunk coating, spin coating, spray coating, puddle dispensing, ultrasonic spray coating, doctor blade coating, aerosol printing, screen printing, microcontact printing, etc. Suitable patterning techniques include photolithography, nanoimprint lithography (NIL), stamping techniques, embossing techniques, molding techniques, microetching techniques, printing techniques, etc. Some examples of suitable resins include a polyhedral oligomeric silsesquioxane based resin (e.g., POSS^{®} from Hybrid Plastics), a non-polyhedral oligomeric silsesquioxane epoxy resin, a poly(ethylene glycol) resin, a polyether resin (e.g., ring opened epoxies), an acrylic resin, an acrylate resin, a methacrylate resin, an amorphous fluoropolymer resin (e.g., CYTOP^{®} from Bellex), and combinations thereof.

As used herein, the term "polyhedral oligomeric silsesquioxane" refers to a chemical composition that is a hybrid intermediate (e.g., RSiO_{1.5}) between that of silica (SiO₂) and silicone (R₂SiO). An example of polyhedral oligomeric silsesquioxane can be that described in Kehagias et al., Microelectronic Engineering 86 (2009), pp. 776-778. In an example, the composition is an organosilicon compound with the chemical formula [RSiO_{3/2}]ₙ, where the R groups can be the same or different. Example R groups for polyhedral oligomeric silsesquioxane include epoxy, azide/azido, a thiol, a poly(ethylene glycol), a norbornene, a tetrazine, acrylates, and/or methacrylates, or further, for example, alkyl, aryl, alkoxy, and/or haloalkyl groups. The resin composition disclosed herein may comprise one or more different cage or core structures as monomeric units.

In an example, the substrate 30, 30' may be fabricated using a round wafer having a diameter ranging from about 2 mm to about 300 mm, or from a rectangular sheet or panel having its largest dimension up to about 10 feet (~ 3 meters). In an example, the substrate 30, 30' is fabricated using a round wafer having a diameter ranging from about 200 mm to about 300 mm. In another example, a panel may be used that is a rectangular support, which has a greater surface area than a 300 mm round wafer. Wafers, panels and other large substrate materials may be diced into the individual flow cell substrates 30, 30'. In another example, the substrate 30, 30' is a die having a width ranging from about 0.1 mm to about 10 mm. While example dimensions have been provided, it is to be understood that a substrate material with any suitable dimensions may be used to fabricate the substrate 30, 30'.

The flow cell 20 also includes flow channels 32. While several flow channels 32 are shown in Fig. 2A, it is to be understood that any number of channels 32 may be included in the flow cell 20 (e.g., a single channel 32, four channels 32 etc.). Each flow channel 32 is an area defined between two bonded components (e.g., the substrate 30, 30' and a lid or two substrates 30, 30'), which can have fluids introduced thereto and removed therefrom. Each flow channel 32 may be isolated from each other flow channel 32 so that fluid introduced into any particular flow channel 32 does not flow into any adjacent flow channel 32. Some examples of the fluids introduced into the flow channels 32 may introduce reaction components (e.g., target genotyping locus library fragments, polymerases, etc.), washing solutions, etc.

As mentioned, the flow channel 32 is defined between the substrate 30, 30' and a lid (not shown) or between the substrate 30, 30' and another substrate (not shown, but similar to substrate 30, 30').

In an example, the lid or additional substrate may be bonded to at least a portion of the substrate 30, 30', e.g., at some of the interstitial regions 40. The bond that is formed between the lid or additional substrate and the substrate 30, 30' may be a chemical bond, or a mechanical bond (e.g., using a fastener, etc.).

The lid may be any material that is transparent to an excitation light that is directed toward the substrate 30, 30'. As examples, the lid may be glass (e.g., borosilicate, fused silica, etc.), plastic, or the like. A commercially available example of a suitable borosilicate glass is D 263^{®}, available from Schott North America, Inc. Commercially available examples of suitable plastic materials, namely cyclo olefin polymers, are the ZEONOR^{®} products available from Zeon Chemicals L.P.

The lid or additional substrate may be bonded to the substrate 30, 30' using any suitable technique, such as laser bonding, diffusion bonding, anodic bonding, eutectic bonding, plasma activation bonding, glass frit bonding, or other methods known in the art. In an example, a spacer layer may be used to bond the lid or additional substrate to the substrate 30, 30'. The spacer layer may be any material that will seal at least some of the substrate 30, 30' and the lid or additional substrate together. In some examples, the spacer layer can be a radiation-absorbing material that aids in bonding.

In an example, the flow channel 32 has a rectangular configuration. The length and width of the flow channel 32 may be smaller, respectively, than the length and width of the substrate 30, 30' so that a portion of the substrate surface surrounding the flow channel 32 is available for attachment to a lid (not shown) or another substrate 30, 30'. In some instances, the width of each flow channel 32 can be at least about 1 mm, at least about 2.5 mm, at least about 5 mm, at least about 7 mm, at least about 10 mm, or more. In some instances, the length of each lane/flow channel 32 can be at least about 10 mm, at least about 25 mm, at least about 50 mm, at least about 100 mm, or more. The width and/or length of each flow channel 32 can be greater than, less than or between the values specified above. In another example, the flow channel 32 is square (e.g., 10 mm x 10 mm).

The depth of each flow channel 32 can be as small as a monolayer thick, for example, when microcontact, aerosol, or inkjet printing is used to deposit the spacer layer that defines the flow channel walls. The depth of the flow channel 32 may be larger, for example, when the flow channel 32 is partially defined in the substrate 30, 30' (e.g., via etching, lithography, etc.) so that a portion of the substrate and the spacer layer define the flow channel walls. For other examples, the depth of each flow channel 32 can be about 1 µm, about 10 µm, about 50 µm, about 100 µm, or more. In an example, the depth may range from about 10 µm to about 100 µm. In another example, the depth may range from about 10 µm to about 30 µm. In still another example, the depth is about 5 µm or less. It is to be understood that the depth of each flow channel 32 can be greater than, less than or between the values specified above.

Referring specifically now to Fig. 2B, an example of the architecture within one of the flow channels 32 of the flow cell 20 is depicted.

As shown in Fig. 2B, the patterned material 36 includes the depressions 38 defined therein, and interstitial regions 40 separating adjacent depressions 38. Many different layouts of the depressions 38 may be envisaged, including regular, repeating, and non-regular patterns. In an example, the depressions 38 are disposed in a hexagonal grid for close packing and improved density. Other layouts may include, for example, rectangular layouts, triangular layouts, and so forth. In some examples, the layout or pattern can be an x-y format of depressions 38 that are in rows and columns. In some other examples, the layout or pattern can be a repeating arrangement of depressions 38 and/or interstitial regions 40. In still other examples, the layout or pattern can be a random arrangement of depressions 38 and/or interstitial regions 40. The pattern may include stripes, swirls, lines, triangles, rectangles, circles, arcs, checks, diagonals, arrows, squares, and/or cross-hatches.

The layout or pattern of the depressions 38 may be characterized with respect to the density of the depressions 38 (number of depressions 38) in a defined area. For example, the depressions 38 may be present at a density of approximately 2 million per mm². The density may be tuned to different densities including, for example, a density of about 100 per mm², about 1,000 per mm², about 0.1 million per mm², about 1 million per mm², about 2 million per mm², about 5 million per mm², about 10 million per mm², about 50 million per mm², or more, or less. It is to be further understood that the density of depressions 38 in the patterned material 36 can be between one of the lower values and one of the upper values selected from the ranges above. As examples, a high density array may be characterized as having depressions 38 separated by less than about 100 nm, a medium density array may be characterized as having depressions 38 separated by about 400 nm to about 1 µm, and a low density array may be characterized as having depressions 38 separated by greater than about 1 µm. While example densities have been provided, it is to be understood that any suitable densities may be used. The density of the depressions 38 may depend, in part, on the depth of the depressions 38. In some instances, it may be desirable for the spacing between depressions 38 to be even greater than the examples listed herein.

The layout or pattern of the depressions 38 may also or alternatively be characterized in terms of the average pitch, or the spacing from the center of the depression 38 to the center of an adjacent depression 38 (center-to-center spacing) or from the left edge of one depression 38 to the right edge of an adjacent depression 38 (edge-to-edge spacing). The pattern can be regular, such that the coefficient of variation around the average pitch is small, or the pattern can be non-regular in which case the coefficient of variation can be relatively large. In either case, the average pitch can be, for example, about 50 nm, about 0.1 µm, about 0.5 µm, about 1 µm, about 5 µm, about 10 µm, about 100 µm, or more or less. The average pitch for a particular pattern of depressions 38 can be between one of the lower values and one of the upper values selected from the ranges above. In an example, the depressions 38 have a pitch (center-to-center spacing) of about 1.5 µm. While example average pitch values have been provided, it is to be understood that other average pitch values may be used.

The size of each depression 38 may be characterized by its volume, opening area, depth, and/or diameter.

Each depression 38 can have any volume that is capable of confining a fluid. The minimum or maximum volume can be selected, for example, to accommodate the throughput (e.g., multiplexity), resolution, nucleotides, or analyte reactivity expected for downstream uses of the flow cell 20. For example, the volume can be at least about 1×10⁻³ µm³, at least about 1×10⁻² µm³, at least about 0.1 µm³, at least about 1 µm³, at least about 10 µm³, at least about 100 µm³, or more. Alternatively or additionally, the volume can be at most about 1×10⁴ µm³, at most about 1×10³µm³, at most about 100 µm³, at most about 10 µm³, at most about 1 µm³, at most about 0.1 µm³, or less.

The area occupied by each depression opening can be selected based upon similar criteria as those set forth above for the volume. For example, the area for each depression opening can be at least about 1×10⁻³ µm², at least about 1×10⁻² µm², at least about 0.1 µm², at least about 1 µm², at least about 10 µm², at least about 100 µm², or more. Alternatively or additionally, the area can be at most about 1×10³ µm², at most about 100 µm², at most about 10 µm², at most about 1 µm², at most about 0.1 µm², at most about 1×10⁻² µm², or less. The area occupied by each depression opening can be greater than, less than or between the values specified above.

The depth of each depression 38 can be large enough to house some of a polymeric hydrogel 42. In an example, the depth may be at least about 0.1 µm, at least about 0.5 µm, at least about 1 µm, at least about 10 µm, at least about 100 µm, or more. Alternatively or additionally, the depth can be at most about 1×10³ µm, at most about 100 µm, at most about 10 µm, or less. In some examples, the depth is about 0.4 µm. The depth of each depression 38 can be greater than, less than or between the values specified above.

In some instances, the diameter or length and width of each depression 38 can be at least about 50 nm, at least about 0.1 µm, at least about 0.5 µm, at least about 1 µm, at least about 10 µm, at least about 100 µm, or more. Alternatively or additionally, the diameter or length and width can be at most about 1×10³ µm, at most about 100 µm, at most about 10 µm, at most about 1 µm, at most about 0.5 µm, at most about 0.1 µm, or less (e.g., about 50 nm). In some examples, the diameter or length and width is about 0.4 µm. The diameter or length and width of each depression 38 can be greater than, less than or between the values specified above.

In the example shown in Fig. 2B, a polymeric hydrogel 42 is positioned within each of the depressions 38. An example of the polymeric hydrogel 42 includes an acrylamide copolymer, such as poly(N-(5-azidoacetamidylpentyl)acrylamide-co-acrylamide, PAZAM. PAZAM and some other forms of the acrylamide copolymer are represented by the following structure (I): 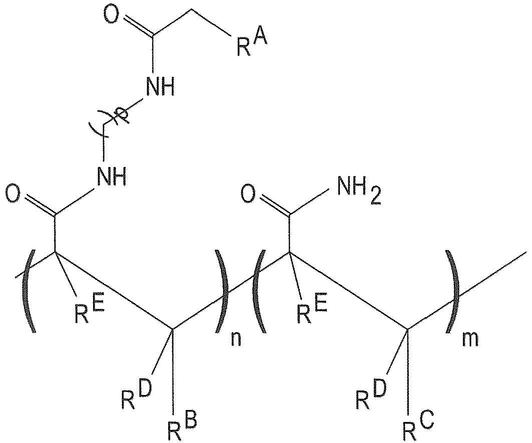 wherein:
R^{A} is selected from the group consisting of azido, optionally substituted amino, optionally substituted alkenyl, optionally substituted alkyne, halogen, optionally substituted hydrazone, optionally substituted hydrazine, carboxyl, hydroxy, optionally substituted tetrazole, optionally substituted tetrazine, nitrile oxide, nitrone, sulfate, and thiol;
R^{B} is H or optionally substituted alkyl;
R^{C,} R^{D}, and R^{E} are each independently selected from the group consisting of H and optionally substituted alkyl;
each of the -(CH₂)ₚ- can be optionally substituted;
p is an integer in the range of 1 to 50;
n is an integer in the range of 1 to 50,000; and
m is an integer in the range of 1 to 100,000.

One of ordinary skill in the art will recognize that the arrangement of the recurring "n" and "m" features in structure (I) are representative, and the monomeric subunits may be present in any order in the polymer structure (e.g., random, block, patterned, or a combination thereof).

The molecular weight of PAZAM and other forms of the acrylamide copolymer may range from about 5 kDa to about 1500 kDa or from about 10 kDa to about 1000 kDa, or may be, in a specific example, about 312 kDa.

In some examples, PAZAM and other forms of the acrylamide copolymer are linear polymers. In some other examples, PAZAM and other forms of the acrylamide copolymer are lightly cross-linked polymers.

In other examples, the polymeric hydrogel 42 may be a variation of the structure (I). In one example, the acrylamide unit may be replaced with N,N-dimethylacrylamide 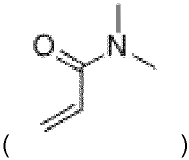 . In this example, the acrylamide unit in structure (I) may be replaced with 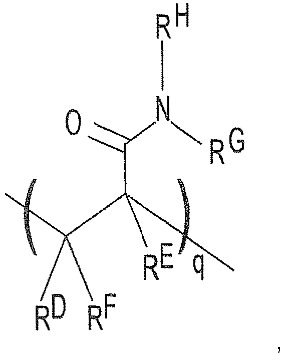 where R^{D}, R^{E}, and R^{F} are each H or a C1-C6 alkyl, and R^{G} and R^{H} are each a C1-C6 alkyl (instead of H as is the case with the acrylamide). In this example, q may be an integer in the range of 1 to 100,000. In another example, the N,N-dimethylacrylamide may be used in addition to the acrylamide unit. In this example, structure (I) may include 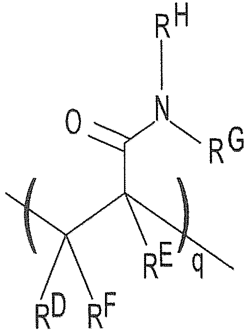 in addition to the recurring "n" and "m" features, where R^{D}, R^{E}, and R^{F} are each H or a C1-C6 alkyl, and R^{G} and R^{H} are each a C1-C6 alkyl. In this example, q may be an integer in the range of 1 to 100,000.

As another example of the polymeric hydrogel 42, the recurring "n" feature in structure (I) may be replaced with a monomer including a heterocyclic azido group having structure (II): 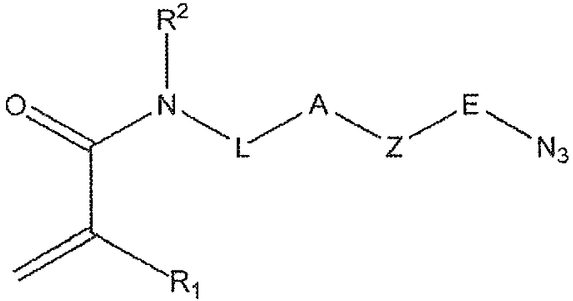 wherein R¹ is H or a C1-C6 alkyl; R₂ is H or a C1-C6 alkyl; L is a linker including a linear chain with 2 to 20 atoms selected from the group consisting of carbon, oxygen, and nitrogen and 10 optional substituents on the carbon and any nitrogen atoms in the chain; E is a linear chain including 1 to 4 atoms selected from the group consisting of carbon, oxygen and nitrogen, and optional substituents on the carbon and any nitrogen atoms in the chain; A is an N substituted amide with an H or a C1-C4 alkyl attached to the N; and Z is a nitrogen containing heterocycle. Examples of Z include 5 to 10 ring members present as a single cyclic structure or a fused structure. Some specific examples of Z include pyrrolidinyl, pyridinyl, or pyrimidinyl.

As still another example, the polymeric hydrogel 42 may include a recurring unit of each of structure (III) and (IV): 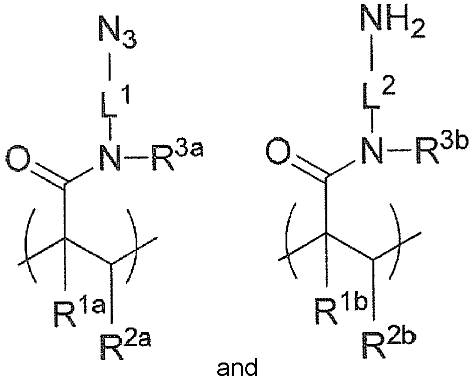 wherein each of R^{1a}, R^{2a}, R^{1b} and R^{2b} is independently selected from hydrogen, an optionally substituted alkyl or optionally substituted phenyl; each of R^{3a} and R^{3b} is independently selected from hydrogen, an optionally substituted alkyl, an optionally substituted phenyl, or an optionally substituted C7-C14 aralkyl; and each L¹ and L² is independently selected from an optionally substituted alkylene linker or an optionally substituted heteroalkylene linker.

It is to be understood that other molecules may be used to form the polymeric hydrogel 42, as long as they are functionalized to graft the capture primers 22, 24 or 22, 24' thereto. Other examples of suitable polymer layers include those having a colloidal structure, such as agarose; or a polymer mesh structure, such as gelatin; or a cross-linked polymer structure, such as polyacrylamide polymers and copolymers, silane free acrylamide (SFA), or an azidolyzed version of SFA. Examples of suitable polyacrylamide polymers may be synthesized from acrylamide and an acrylic acid or an acrylic acid containing a vinyl group, or from monomers that form [2+2] photo-cycloaddition reactions. Still other examples of suitable polymeric hydrogels 42 include mixed copolymers of acrylamides and acrylates. A variety of polymer architectures containing acrylic monomers (e.g., acrylamides, acrylates etc.) may be utilized in the examples disclosed herein, such as branched polymers, including star polymers, star-shaped or star-block polymers, dendrimers, and the like. For example, the monomers (e.g., acrylamide, etc.) may be incorporated, either randomly or in block, into the branches (arms) of a star-shaped polymer.

To introduce the polymeric hydrogel 42 into the flow channel 32, a mixture of the polymeric hydrogel 42 may be generated and then applied to the substrate 30, 30' (including the depressions 38). In one example, the polymeric hydrogel 42 may be present in a mixture (e.g., with water or with ethanol and water). The mixture may then be applied to the substrate surfaces (including in the depressions 38) using spin coating, or dipping or dip coating, spray coating, or flow of the material under positive or negative pressure, or another suitable technique. These types of techniques blanketly deposit the polymeric hydrogel 42 on the substrate 30, 30' (e.g., in the depressions 38 and on interstitial regions 40 surrounding the depressions 38). Other selective deposition techniques (e.g. involving a mask, controlled printing techniques, etc.) may be used to specifically deposit the polymeric hydrogel 42 in the depressions 38 and not on the interstitial regions 40.

In some examples, the substrate surface (including the portion that is exposed in the depressions 38) may be activated, and then the mixture (including the polymeric hydrogel 42) may be applied thereto. In one example, a silane or silane derivative (e.g., norbornene silane) may be deposited on the substrate surface using vapor deposition, spin coating, or other deposition methods. In another example, the substrate surface may be exposed to plasma ashing to generate surface-activating agent(s) (e.g., -OH groups) that can adhere to the polymeric hydrogel 42.

Depending upon the polymeric hydrogel 42, the applied mixture may be exposed to a curing process. In an example, curing may take place at a temperature ranging from room temperature (e.g., from about 18°C to about 25°C) to about 95°C for a time ranging from about 1 millisecond to about several days.

In some examples, polishing may then be performed in order to remove the polymeric hydrogel 42 from the interstitial regions 40 at the perimeter of the depressions 38, while leaving the polymeric hydrogel 42 on the surface in the depressions 38 at least substantially intact.

The flow cell 20 also includes the first and second capture primers 22, 24 or 22, 24'. Any example of the capture primers 22, 24 or 22, 24' set forth herein may be used. The set of capture primers 22, 24 or 22, 24' that is selected for a particular flow cell 20 may depend, in part, upon which genotyping oligonucleotide 10, 10' is to be used therewith.

A grafting process may be performed to graft the first and second capture primers 22, 24 or 22, 24' to the polymeric hydrogel 42 in the depressions 38. In an example, the first and second capture primers 22, 24 or 22, 24' can be immobilized to the polymeric hydrogel 42 by single point covalent attachment at or near the 5' ends of each of the first and second capture primers 22, 24 or 22, 24'. This attachment leaves i) a primer sequence-specific portion of the capture primers 22, 24 or 22, 24' free to anneal to its cognate primer sequence 12 or copied primer sequence C₁₈, C_{18'}, and ii) a 3' hydroxyl (OH) group free for capture primer extension. Any suitable covalent attachment may be used for the attachment of the first and second capture primers 22, 24 or 22, 24' to the polymeric hydrogel 42. Examples of terminated primers that may be used include alkyne terminated primers, which can attach to an azide moiety of the polymeric hydrogel 42. As mentioned, specific examples of suitable capture primers 22, 24 include the P5 and P7 primers, and a specific example of a suitable capture primer 24' is P7 modified to include the second restriction endonuclease site.

In an example, grafting may involve flow through deposition (e.g., using a temporarily bound or permanently bonded lid or additional substrate), dunk coating, spray coating, puddle dispensing, or by another suitable method that will attach the capture primer(s) 22, 24 or 22, 24' to the polymeric hydrogel 42. Each of these example techniques may utilize a primer solution or mixture, which may include the capture primer(s) 22, 24 or 22, 24', water, a buffer, and a catalyst. With any of the grafting methods, the capture primer(s) 22, 24 or 22, 24' react with reactive groups of polymeric hydrogel 42 in the depressions 38 and have no affinity for the surrounding interstitial regions 40. As such, the capture primer(s) 22, 24 or 22, 24' selectively graft to the polymeric hydrogel 42 in the depressions 38.

### Methods Involving the Genotyping Oligonucleotides

Examples of the method disclosed herein generally include introducing a genotyping probe fluid to a flow cell 20 including individual depressions 38 and first and second capture primers 22, 24 or 22, 24' in each of the individual depressions 38, the genotyping probe fluid including a plurality of genotyping oligonucleotides 10 or 10', whereby a respective genotyping oligonucleotide 10 or 10' reacts in at least some of the individual depressions 38 to produce respective clonal populations of amplicons from the respective genotyping oligonucleotide 10 or 10'; linearizing the amplicons to produce probe templates; sequencing at least one probe identifying section of the probe templates to identify each of the probe sequences; removing at least respective nascent strands from the probe templates, whereby a 3' OH group at an end of the probe templates is exposed; hybridizing respective samples to the probe templates; and performing respective genotyping reactions of the samples at the exposed 3' OH groups.

In examples of the method, the flow cell 20 may be introduced into a system (not shown), where it is in fluid communication with a fluidic control system (e.g., pumps, valves, and the like) and is in optical communication with an illumination system and a detection system.

Fig. 3A through Fig. 3H together illustrate an example of the method involving the genotyping oligonucleotide 10.

Fig. 3A depicts one depression 38 of the flow cell 20, which includes the polymeric hydrogel 42 having the first and second capture primers 22, 24 attached thereto. As described herein, the second capture primer 24 includes a cleavage site 46.

In Fig. 3B, a genotyping probe fluid (not shown) is introduced into the flow cell 20 (e.g., into each flow channel 32). In this example, the genotyping probe fluid includes a liquid carrier and the genotyping oligonucleotide 10 in the liquid carrier. The liquid carrier of the genotyping probe fluid may be any suitable hybridization buffer, such as Tris-HCl buffer or 0.5x saline sodium citrate (SSC) buffer. In some examples, the genotyping probe fluid includes a plurality of genotyping oligonucleotides 10, where each genotyping oligonucleotide 10 includes a different probe sequence 14 than each other genotyping oligonucleotide 10. With this fluid, different genotyping oligonucleotides 10 (each with a unique probe sequence 14) can be delivered to different depressions 38.

As shown in Fig. 3B, one genotyping oligonucleotide 10 is seeded within the depression 38. More specifically, the second primer sequence 18 of one genotyping oligonucleotide 10 hybridizes to one of the second capture primers 24 in the depression 38.

When one genotyping oligonucleotide 10 is seeded, cluster generation may be immediately initiated. In other examples, separate hybridization (seeding) and cluster generation may be performed. The processes involved in cluster generation are shown in Fig. 3B, Fig. 3C, Fig. 3D, and Fig. 3E.

As represented by the arrow in Fig. 3B, the genotyping oligonucleotide 10 is copied from the hybridized primers by 3' extension using a DNA polymerase. This generates the amplicon 44A, which is attached to the flow cell surface through the second capture primer 24. The sections labeled C₁₆, C₁₄, C₁₂ of the amplicon 44A are complementary copies of the restriction endonuclease site 16, the probe sequence 14, and the first primer sequence 12, respectively.

The original genotyping oligonucleotide 10 is denatured, leaving the amplicon 44A immobilized in the depression 38 via the second capture primer 24. The single-stranded amplicon 44A flips over and forms a bridge, e.g., by the hybridization of the first primer sequence copy C₁₂ to an adjacent, complementary first capture primer 22. This is shown in Fig. 3C. As represented by the arrow in Fig. 3C, the hybridized primer (first capture primer 22) is then extended by polymerase(s) to form another amplicon 44B. The sections CC₁₆, CC₁₄ of the amplicon 44B are complementary copies of the sections C₁₆, C₁₄, and thus have the same sequences as the original restriction endonuclease site 16 and probe sequence 14, respectively. The section C₂₄ of the amplicon 44B is a complementary copy of the second capture primer 24, and thus has the same sequence as the second primer sequence 18. As depicted in Fig. 3C, the formation of the amplicon 44B generates a double stranded bridge including the amplicons 44A and 44B.

The double stranded bridge is then denatured, as shown in Fig. 3D. This results in two copies (amplicons 44A and 44B) that are covalently bound to the flow cell 20. Isothermal bridge amplification or some other form of amplification amplifies the immobilized copies. For example, the copied templates loop over to hybridize to an adjacent, complementary capture primer 22, 24, and a polymerase copies the copied templates to form double stranded bridges, which are denatured to form two single stranded strands. These two strands loop over and hybridize to adjacent, complementary capture primers 22, 24 and are extended again to form two new double stranded loops. The process is repeated on each template copy by cycles of isothermal denaturation and amplification to create dense clonal clusters of double stranded bridges. A simplified cluster, including two double stranded bridges, is shown in Fig. 3E.

It is to be understood that the seeding of respective genotyping oligonucleotides 10 in respective depressions 38 and the amplification of such genotyping oligonucleotides 10 in the respective depressions 38 can take place under conditions where the amplification rate exceeds the seeding rate. As such, the relatively rapid rate at which copies (amplicons 44A, 44B) are made within the depression 38 that has been seeded with one genotyping oligonucleotide 10 will effectively exclude a second genotyping oligonucleotide 10 from seeding within that depression 38 for amplification. As such, a different genotyping oligonucleotide 10 (with a unique probe sequence 14) can be captured and amplified in each of the depressions 38, which enables a plurality of different target genotyping loci to be analyzed simultaneously on the flow cell 20.

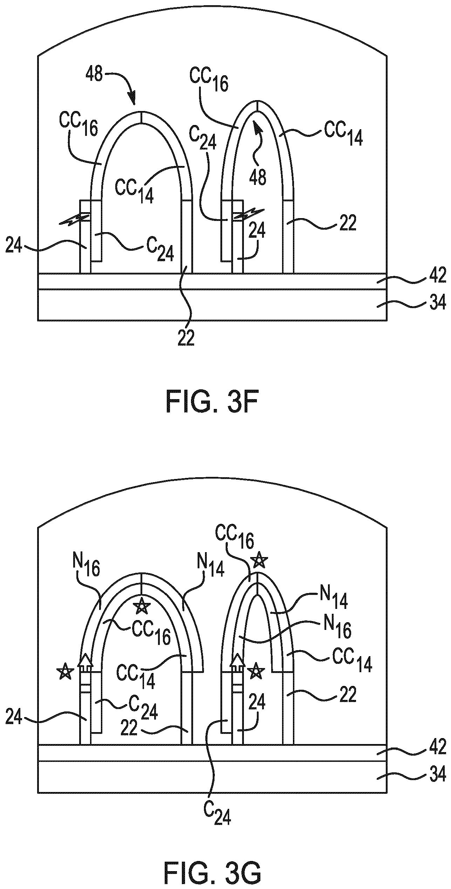

Linearization of the bridged amplicons 44A, 44B may be performed by cleaving the amplicons attached to the second capture primers (e.g., amplicons 44A) at respective cleavage sites 46 of the second capture primers 24; and denaturing cleaved portions of the amplicons (e.g., amplicons 44A) attached to the second capture primers 24 to produce the probe templates 48 (Fig. 3F).

To initiate cleavage, a cleaving agent may be introduced into the flow cell 20, e.g., through an input port (not shown). The cleaving agent that is selected will depend upon the cleavage site 46 of the second capture primer 24. The cleaving agent may be a chemical cleaving agent or an enzymatic cleaving agent depending on the cleavage site 46. Cleavage at the cleavage site 46 severs the amplicons 44A between the second capture primers 24 and the remainder of the amplicon sequence (which includes sections C₁₆, C₁₄, and C₁₂ or sections C₁₆, C₁₄, and C₂₂ (which is a complementary copy of the capture primer 22)).

As a result of the cleavage, the sections C₁₆, C₁₄, and C₁₂ and the sections C₁₆, C₁₄, and C₂₂ are no longer attached to the flow cell surface through a primer 24, and thus can be removed via denaturation. Denaturation may take place using any suitable conditions. The removal of the sections C₁₆, C₁₄, and C₁₂ and the sections C₁₆, C₁₄, and C₂₂ leaves the amplicons 44B attached to the flow cell surface through the first capture primer 22 and hybridized to the second capture primers 24 (through section C₂₄). In this example, the exposed single stranded portion of the amplicons 44B, specifically sections CC₁₆ and CC₁₄, make up the probe template 48. The section CC₁₄ is a complementary copy of the section C₁₄, and thus has the same sequence as the probe sequence 14. Sequencing this section CC₁₄ enables the original probe sequence 14 to be identified/decoded. In some instances, a portion of the section CC₁₄ may be sequenced to identify or decode the original probe sequence. The section CC₁₆ is a complementary copy of the section C₁₆, and thus has the same sequence as the restriction enzyme site 16. When sequenced, the double stranded section including CC₁₆ and N₁₆ (Fig. 3G) provides a substrate for the restriction enzyme cleavage.

After cleavage and denaturation, each second capture primer 24 may have a 3' phosphate at its end that should be removed prior to additional processing. The 3' phosphate may be removed by introduction of a kinase, which deprotects the second capture primer 24, rendering it suitable for use as a sequencing primer (as described in reference to Fig. 3G).

Fig. 3G illustrates the sequencing of the probe template 48, including the sections CC₁₆ and CC₁₄, the latter of which is the at least one probe identifying section.

In the example shown, sequencing the probe template 48 involves using the second capture primer 24 as a sequencing primer, and performing a base extension reaction (one base at a time) along the probe template 48.

In another example, the section C₂₄ and the second capture primer 24 may be denatured, and a separate sequencing primer (that is in solution) may be added. The separate sequencing primer may hybridize to the section C₂₄, and a base extension reaction (one base at a time) would be performed along the probe template 48.

The underlying chemical process for sequencing can be polymerization (e.g., catalyzed by a polymerase enzyme). In a particular polymerase-based process, fluorescently labeled nucleotides are added to the second capture primer 24 in a template dependent fashion such that detection of the order and type of nucleotides added to the second capture primer 24 can be performed. This enables one to determine the sequence of the probe identifying section, e.g., section CC₁₄, which can be used to decode the original probe sequence 14.

To initiate a first sequencing cycle, one or more labeled nucleotides, DNA polymerase, etc., may be delivered into/through the flow cell 20 etc., where sequencing primer extension causes a labeled nucleotide to be incorporated to the probe template 48. This incorporation can be detected through an imaging event. During an imaging event, an illumination system may provide an excitation light to the flow cell 20.

In some examples, the fluorescently labeled nucleotides can further include a reversible termination property that terminates further primer extension once a nucleotide has been added to the template 48. For example, a nucleotide analog having a reversible terminator moiety can be added to the template 48 such that subsequent extension cannot occur until a deblocking agent is delivered to remove the moiety. Thus, for examples that use reversible termination, a deblocking reagent can be delivered to the flow cell 20, etc. (after detection occurs).

Wash(es) may take place between the various fluid delivery steps. The sequencing cycle can then be repeated n times to extend the template 48 by n nucleotides to generate a nascent strand including the nascent section N₁₆ (which is complementary to section CC₁₆) and the nascent section N₁₄ (which is complementary to section CC₁₄).

Sequencing the section CC₁₄ provides the nascent strand section N₁₄, which can be used to decode the original probe sequence 14 of the genotyping oligonucleotide 10. This information allows the user to identify the target genotyping locus that will be analyzed in a particular depression 38 (since all of the templates 48 in a depression 38 have the same probe identifying section, e.g., CC₁₄).

Sequencing the section CC₁₆ provides the nascent strand section N₁₆. As such, this example of sequencing also generates the double stranded section, including both CC₁₆ and N₁₆, which provides a substrate for the restriction enzyme cleavage.

After sequencing the probe identifying section(s), the method further includes removing at least respective nascent strands (which include sections N₁₄ and N₁₆) from the probe templates 48, whereby a 3' OH group at an end of the probe templates 48 is exposed. In this example, removal involves more than the removal of the nascent strands N₁₄ and N₁₆. In this example, removing involves digesting the restriction endonuclease sites, e.g., sections CC₁₆ and N₁₆, and denaturing the remaining nascent strand, including section N₁₄, from the probe templates 48.

The probe template 48 includes the section CC₁₆ (which has the same sequence as the restriction endonuclease site 16) and the nascent strand includes the section N₁₆ (which is complementary to the restriction endonuclease site 16). This double stranded portion (sections CC₁₆ and N₁₆) creates a substrate for an appropriate restriction endonuclease (restriction enzyme). As such, by introduction of the restriction endonuclease, the restriction endonuclease sites, in this example, section CC₁₆ and section N₁₆, can be digested. As mentioned herein, the restriction enzyme may be a 4 base cutter restriction endonuclease, a 5 base cutter restriction endonuclease, a 6 base cutter restriction endonuclease, or the like. The restriction enzyme cleaves the sections CC₁₆, N₁₆ at specific nucleotides, which are identified schematically by the stars in Fig. 3G. In this example, restriction endonuclease site digestion leaves the second capture primer 24, and the first capture primer 22 having the section CC₁₄ attached thereto, and the nascent strand N₁₄ hybridized to the section CC₁₄. The nascent strand N₁₄ is then denatured from the section CC₁₄. Digestion and denaturation enables the section CC₁₆ and the nascent strands N₁₄, N₁₆ to be removed from the depression 38 and flow cell 20, e.g., via a washing step.

Digestion and denaturation exposes a 3' OH at an end of what remains of the probe template 48. The remaining probe template is shown at reference numeral 52 in Fig. 3H. The remaining probe template 52 includes the first capture primer 22 and the section CC₁₄, which is the same as the original probe sequence 14, and thus, in this example, is complementary to a DNA sample having the target genotyping locus 54.

A sample of denatured DNA fragments, including the target genotyping locus 54, is introduced into the flow cell 20. The target genotyping locus 54 may be included in a library fluid that includes a plurality of target genotyping loci, at least some of which have different loci to be genotyped. The target genotyping loci may be prepared from the larger DNA sample using any genotyping library preparation technique. Some genotyping library preparation techniques involve amplification and fragmentation. Others involve amplification without fragmentation (examples of which are described hereinbelow). As such, several copies of any one type of target genotyping locus 54 may be present in the library fluid.

Once introduced into the flow cell 20, the target genotyping loci 54 hybridize to respective complementary sections CC₁₄ of remaining probe templates 52 in the depression 38.

The genotyping reaction may then be performed. In this reaction, the remaining probe template 52 is used as a sequencing primer to perform one cycle of sequencing as described herein. As shown in Fig. 3H, one labeled nucleotide 57, which is complementary to the nucleobase of interest on the target genotyping locus 54, is incorporated into the remaining probe template 52.

While the description herein is directed to one depression 38 and thus genotyping one target genotyping locus 54, it is to be understood that each depression 38 includes different probe templates 52 with different sections CC₁₄. As such, with this method, hundreds to thousands to millions (depending on the number of depressions in the flow cell 20) of different loci can be genotyped simultaneously.

Fig. 4A through Fig. 4H together illustrate another example of the method involving the genotyping oligonucleotide 10'.

Fig. 4A depicts one depression 38 of the flow cell 20, which includes the polymeric hydrogel 42 having the first and second capture primers 22, 24' attached thereto. As described herein, the second capture primer 24' includes the second restriction endonuclease site 46'.

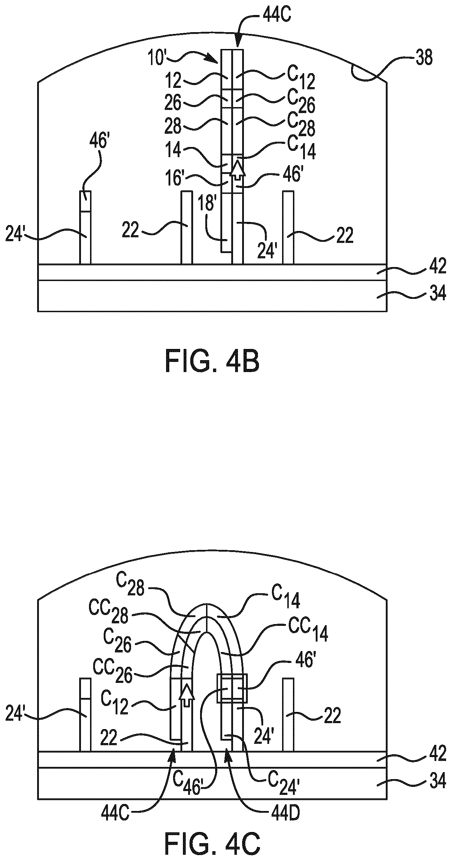

In Fig. 4B, a genotyping probe fluid (not shown) is introduced into the flow cell 20 (e.g., into each flow channel 32). In this example, the genotyping probe fluid includes a liquid carrier and the genotyping oligonucleotide 10' in the liquid carrier. The liquid carrier may be any of the examples disclosed herein. In some examples, the genotyping probe fluid includes a plurality of genotyping oligonucleotides 10', where each genotyping oligonucleotide 10' includes a different probe sequence 14 than each other genotyping oligonucleotide 10'. With this fluid, different genotyping oligonucleotides 10' (each with a unique probe sequence 14) can be delivered to different depressions 38.

As shown in Fig. 4B, one genotyping oligonucleotide 10' is seeded within the depression 38. More specifically, the second primer sequence 18' and the restriction endonuclease site 16' of one genotyping oligonucleotide 10' respectively hybridize to one of the second capture primers 24' and its restriction endonuclease site 46' in the depression 38.

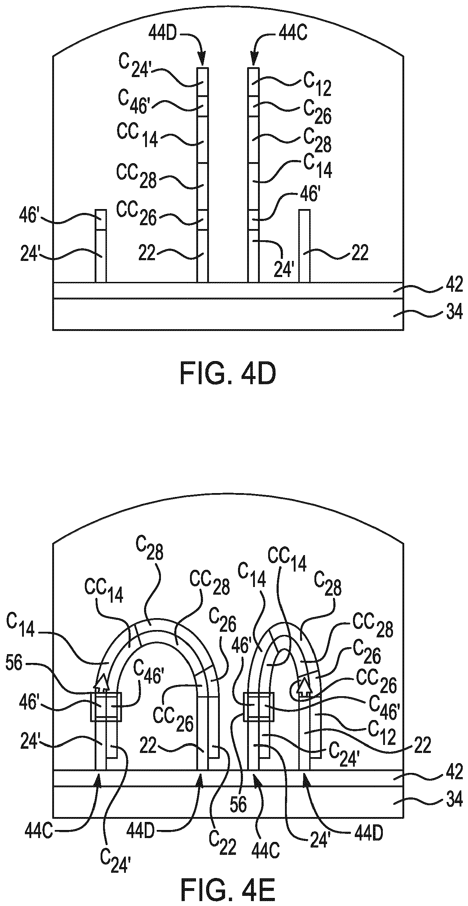

When one genotyping oligonucleotide 10' is seeded, cluster generation may be immediately initiated. In other examples, separate hybridization (seeding) and cluster generation may be performed. The processes involved in cluster generation are shown in Fig. 4B, Fig. 4C, Fig. 4D, and Fig. 4E.

As represented by the arrow in Fig. 4B, the genotyping oligonucleotide 10' is copied from the hybridized primers by 3' extension using a high-fidelity DNA polymerase. This generates the amplicon 44C, which is attached to the flow cell surface through the second capture primer 24'. The sections labeled C₁₄, C₂₈, C₂₆, and C₁₂ of the amplicon 44C are complementary copies of the probe sequence 14, the priming site portion 28, the index sequence portion 26, and the first primer sequence 12, respectively.

The original genotyping oligonucleotide 10' is denatured, leaving the amplicon 44C immobilized in the depression 38 via the second capture primer 24' and the second restriction endonuclease site 46'. The single-stranded amplicon 44C flips over and forms a bridge, e.g., by the hybridization of the first primer sequence copy C₁₂ to an adjacent, complementary first capture primer 22. This is shown in Fig. 4C.

As represented by the arrow in Fig. 4C, the hybridized primer is then extended by polymerase(s) to form another amplicon 44D. The sections CC₁₄, CC₂₆, CC₂₈ of the amplicon 44D are complementary copies of the sections C₁₄, C₂₆, C₂₈, and thus have the same sequences as the original probe sequence 14, index sequence portion 26, and priming site portion 28, respectively. The section C_{46'} of the amplicon 44D is a complementary copy of the second restriction endonuclease site 46', and thus has the same sequence as the restriction endonuclease site 16' of the genotyping oligonucleotide 10'. The section C_{24'} of the amplicon 44D is a complementary copy of the second capture primer 24', and thus has the same sequence of the second primer sequence 18'. As depicted in Fig. 4C, the formation of the amplicon 44D generates a double stranded bridge including the amplicons 44C and 44D covalently bound to the flow cell 20.

The double stranded bridge is then denatured, as shown in Fig. 4D. This results in two copies (amplicons 44C and 44D) that are covalently bound to the flow cell 20. Isothermal bridge amplification or some other form of amplification amplifies the immobilized copies. For example, the copied templates loop over to hybridize to an adjacent, complementary capture primer 22, 24', and a polymerase copies the copied templates to form double stranded bridges, which are denatured to form two single stranded strands. These two strands loop over and hybridize to adjacent, complementary capture primers 22, 24' and are extended again to form two new double stranded loops. The process is repeated on each template copy by cycles of isothermal denaturation and amplification to create dense clonal clusters of double stranded bridges. A simplified cluster, including two double stranded bridges, is shown in Fig. 4E.

It is to be understood that the seeding of respective genotyping oligonucleotides 10' in respective depressions 38 and the amplification of such genotyping oligonucleotides 10' in the respective depressions 38 can take place under conditions where the amplification rate exceeds the seeding rate. As such, the relatively rapid rate at which copies (amplicons 44C, 44D) are made within the depression 38 that has been seeded with one genotyping oligonucleotide 10' will effectively exclude a second genotyping oligonucleotide 10' from seeding within that depression 38 for amplification. As such, a different genotyping oligonucleotide 10' (with a unique probe sequence 14) can be captured and amplified in each of the depressions 38, which enables a plurality of different target genotyping loci to be analyzed simultaneously on the flow cell 20.

In this example, amplification may be performed using methylated dCTP (deoxycytidine triphosphate) to protect the restriction endonuclease site 16' and complementary copies C_{46'} of the second restriction endonuclease site 46'. This modification will fully methylate the amplicons 44C, 44D and leave the capture primers 22, 24' (including the second restriction endonuclease site 46') hemi-methylated.

The double stranded bridges are then linearized. Linearization is described in reference to Fig. 4E and Fig. 4F. Linearization of the bridged amplicons 44C, 44D in this example method may be performed by digesting the restriction endonuclease portion 56 of the amplicons 44C, 44D to leave the second capture primers 24' in the depressions 38; and denaturing a remaining portion of the amplicons 44C, 44D to produce single stranded probe templates 49 including the first capture primers 22 and the at least one probe identifying section in the depressions 38. The result of the linearization process is shown in Fig. 4F.

In this example, each of the second capture primers 24' further includes the second restriction endonuclease site 46', wherein the second restriction endonuclease site 46' is complementary to the restriction endonuclease site 16' of the genotyping oligonucleotide 10' and thus is also complementary to the section C_{46'}. As shown in Fig. 4E, the double stranded bridges include the hybridized sections 46', C_{46'}, which provide respective substrates for restriction enzyme cleavage. More specifically, the hybridized sections C₄₆, and 46' of the bridged amplicons 44C, 44D make up restriction endonuclease portion 56, which is recognizable by a Type IIS restriction enzyme. In this example, the digestion of the restriction endonuclease portion 56 is accomplished by the introduction of the Type IIS restriction enzyme. The Type IIS restriction enzyme may be methyl sensitive or may not be methyl sensitive. Methylated protocols will protect sites internal to the probe sequence copies C₁₄, CC₁₄ and/or may protect symmetric cuts. The Type IIS restriction enzyme will recognize the asymmetric DNA sequences of the portion 56 and cleave at a defined distance (e.g., from 1 nucleotide to about 20 nucleotides) outside of the portion 56. Digestion leaves the second capture primers 24' attached to the flow cell 20, and also cleaves the amplicon 44C at a desirable position for genotyping.

The remaining portions of the amplicons 44C, 44D may then be denatured, which leaves the single stranded probe templates 49 attached to the flow cell 20. As shown in Fig. 4F, the single stranded probe templates 49 include the first capture primers 22 and the at least one probe identifying section, which in this example includes some or all of section CC₁₄, as well as sections CC₂₈ and CC₂₆.

The method may further comprise blocking the second capture primers 24' prior to sequencing along the at least one probe identifying section of each of the first single stranded probe templates 49. A blocking group (e.g., a 3' phosphate) may be added that attaches to the exposed 3' ends of the second capture primers 24' to prevent undesired extension at these primers 24'.

Referring now to Fig. 4F, the sequencing of at least one probe identifying section of the single stranded probe templates 49 is depicted. In this example, the at least one probe identifying section is the section CC₂₆, which is identical to the index sequencing portion 26.

Sequencing the at least one probe identifying section involves introducing a sequencing primer 50, which hybridizes to the section CC₂₈, which is identical to the priming site portion 28. This sequencing primer 50 renders the at least one probe identifying section, e.g., CC₂₆, of the single stranded probe template 49 ready for sequencing. A base extension reaction is then performed along the section CC₂₆.

The underlying chemical process for sequencing the can be polymerization (e.g., catalyzed by a polymerase enzyme) as described herein in reference to Fig. 3G.

To initiate a first sequencing cycle, one or more labeled nucleotides, DNA polymerase, etc., may be delivered into/through the flow cell 20 etc., where sequencing primer extension causes a labeled nucleotide to be incorporated into a nascent strand N₂₆ formed along the section CC₂₆ of the single stranded probe template 49. This incorporation can be detected through an imaging event.

In some examples, the fluorescently labeled nucleotides can further include a reversible termination property that terminates further primer extension once a nucleotide has been added to the nascent strand N₂₆. Thus, for examples that use reversible termination, a deblocking reagent can be delivered to the flow cell 20, etc. (after detection occurs).

Wash(es) may take place between the various fluid delivery steps. The sequencing cycle can then be repeated n times to extend the single stranded probe template 49 by n nucleotides to generate a nascent strand including the nascent section N₂₆ (which is complementary to section CC₂₆, which has the same sequence as the index sequencing portion 26).

Sequencing of the nascent strand N₂₆ can be used to identify the section CC₂₆, and thus the original index sequencing portion 26, which is unique to the probe sequence 14 (and its complementary copy C₁₄). This information allows the user to identify the target genotyping locus that will be analyzed in a particular depression 38 (since all of the templates 49 in a depression 38 have the same probe identifying section, e.g., CC₂₆, and probe sequence section, e.g., CC₁₄).

After sequencing the probe identifying section(s), the method further includes removing at least respective nascent strands (which includes section N₂₆) from the single stranded probe templates 49. In this example, removing involves denaturing the sequencing primer 50 and the nascent strand N₂₆.

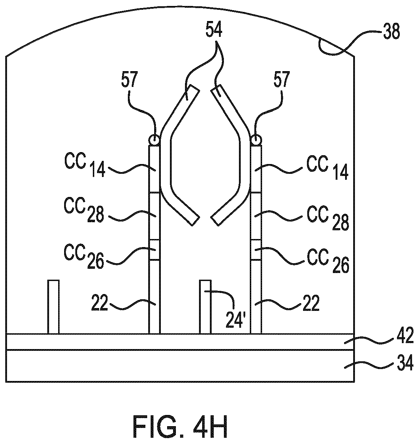

A sample of single stranded DNA fragments, including the target genotyping locus 54, is introduced into the flow cell 20, as shown in Fig. 4H. The target genotyping locus 54 may be included in a library fluid that includes a plurality of target genotyping loci, at least some of which have different loci to be genotyped. The target genotyping loci may be prepared from the larger DNA sample using any genotyping library preparation technique. Some genotyping library preparation techniques involve amplification and fragmentation. Others involve amplification without fragmentation, as described hereinbelow. As such, several copies of any one type of target genotyping locus 54 may be present in the library fluid.

Once introduced into the flow cell 20, the target genotyping loci 54 hybridize to respective complementary sections CC₁₄ of the single stranded probe templates 49 in the depression 38.

The genotyping reaction may then be performed. In this reaction, the single stranded probe template 49 is used as a sequencing primer to perform one cycle of sequencing as described herein. As shown in Fig. 4H, one labeled nucleotide 57, which is complementary to the nucleobase of interest on the target genotyping locus 54, is incorporated into the single stranded probe template 49.

While the description herein is directed to one depression 38 and thus genotyping one target genotyping locus 54, it is to be understood that each depression 38 includes different single stranded probe templates 49 with different sections CC₁₄. As such, with this method, hundreds to thousands to millions (depending on the number of depressions in the flow cell 20) of different loci can be genotyped simultaneously.

In the examples disclosed herein, instead of blocking the second capture primers 24 or 24' during sequencing and/or genotyping, these primers 24 or 24' could be cleaved following the de-coding reactions using a cleaving process that will not deleteriously affect the templates 48 or 49 to be genotyped.

### Genotyping Library Preparation Technique

Any suitable genotyping library preparation technique may be used to prepare the target genotyping locus 54.

The target genotyping locus 54 may be prepared from genomic DNA. Genomic DNA can be isolated from one or more cells, bodily fluids or tissues. Any suitable method can be used to obtain a bodily fluid (e.g., blood, sweat, tears, lymph, urine, saliva, semen, cerebrospinal fluid, feces or amniotic fluid). Some specific examples include a buccal swab, mouthwash, surgical removal, biopsy aspiration or the like. Genomic DNA can also be obtained from one or more cell or tissue in primary culture, in a propagated cell line, a fixed archival sample, forensic sample or archeological sample.

gDNA can be prepared by lysing a cell that contains the DNA. The cell may be lysed under conditions that substantially preserve the integrity of the cell's gDNA. In one particular example, thermal lysis may be used to lyse a cell. In another particular example, exposure of a cell to alkaline pH can be used to lyse a cell while causing relatively little damage to gDNA. Any of a variety of basic compounds can be used for lysis including, for example, potassium hydroxide, sodium hydroxide, and the like. Additionally, relatively undamaged gDNA can be obtained from a cell lysed by an enzyme that degrades the cell wall. Cells lacking a cell wall either naturally or due to enzymatic removal can also be lysed by exposure to osmotic stress. Other conditions that can be used to lyse a cell include exposure to detergents, mechanical disruption, sonication heat, pressure differential such as in a French press device, or Dounce homogenization. Agents that stabilize gDNA can be included in a cell lysate or isolated gDNA sample including, for example, nuclease inhibitors, chelating agents, salts, buffers and the like.

In some examples, a crude cell lysate containing gDNA can be directly amplified without further isolation of the gDNA. For example, a blood sample may be exposed to thermal lysis, and then the crude cell lysate may be amplified using any suitable method, including those described herein. Alternatively, gDNA can be further isolated from other cellular components prior to amplification. Accordingly, amplification can be carried out on purified or partially purified gDNA. Genomic DNA can be isolated using known methods including, for example, liquid phase extraction, precipitation, solid phase extraction, chromatography and the like.

An amplified representative population of genome fragments (target genotyping loci 54) can be provided by amplifying a native genome under conditions that replicate the genomic DNA (gDNA) template to produce one or more copies in which the relative proportion of each copied sequence is substantially the same as its proportion in the original gDNA. Any of a variety of methods that replicate genomic DNA in a sequence independent fashion can be used to prepare the target genotyping loci 54. In the examples disclosed herein, double stranded genomic DNA may be denatured to generate single stranded genomic DNA templates that can be used in the amplification processes.

In one specific example, the amplification method involves contacting a single stranded genomic DNA template with a low processivity polymerase, a plurality of primers, and free nucleotides, thereby generating complementary fragments of the single stranded genomic DNA template; and displacing the complementary fragments from the single stranded genomic DNA template, thereby generating at least some of the respective samples.

The low processivity polymerase can synthesize short strands because they naturally fall off of the single stranded genomic DNA template before the entire strand is replicated. Some examples of the low processivity polymerase can synthesize less than 100 bases per polymerization event. Shorter fragments can be obtained by using a polymerase that synthesizes less than 50, 40, 30, 20, 10 or 5 bases per polymerization event under the conditions of amplification. The low processivity polymerase is selected from the group consisting of T4 DNA polymerase, T7 DNA polymerase, Taq polymerase, Stoffel Fragment (fragment of Taq DNA polymerase), Klenow Fragment (the large fragment of Escherichia coli DNA polymerase I), Bsu DNA polymerase, Bst DNA polymerase, and an engineered polymerase. In this example method, the term "engineered polymerase" is any synthetic polymerase that is designed to synthesize less than 100 bases per polymerization event. Other suitable low processivity polymerases include monomeric *E*. *coli* Pol III (lacking the beta subunit) or *E*. *coli* Pol I.

The processivity of some polymerases may be altered by the processing conditions that are used. For example, the processivity may be altered by the temperature of the reaction, the salt (e.g., Mg²⁺) level in the reaction (e.g., ionic strength), the pH, the buffer composition (e.g., creatine kinase or cAMP (cyclic adenosine monophosphate)), or combinations thereof. As one example, a T7 DNA polymerase has low processivity at temperatures below 37°C, and also at high ionic strengths, e.g., greater than about 100 mM NaCl; but otherwise has high processivity. As another example, a Taq polymerase has high processivity at temperatures around 70°C when reacted with a 10 fold molar excess of the DNA samples and the random primers. In another example, Klenow fragment of Escherichia coli DNA polymerase polymerization can be slowed by lowering the pH to below pH 6.2. In still another example, the efficiency of BST DNA polymerase (BST pol), an A family DNA pol, can be manipulated by several fold through the substitution of metal co-factors such as Mg++ and Cd++.

The primers may be random primers. A population of random primers can be synthesized to include a higher content of guanine (G) and/or cytosine (C) nucleotides compared to adenine (A) and thymidine (T) nucleotides. The resulting random primer population will be GC rich and therefore have a higher probability of hybridizing to high GC regions of a genome such as gene coding regions of a human genome which typically have a higher GC content than non-coding gDNA regions. Primers in a population of random primers can also have a region of identical sequence such as a universal tail. A universal tail can include a universal priming site for amplification.

In this example method, the free nucleotides may include any natural nucleotide, such as deoxyadenine triphosphate, deoxythymine triphosphate, deoxyguanine triphosphate, and deoxycytosine triphosphate.

Contacting the single stranded genomic DNA template with the low processivity polymerase, the plurality of primers, and the free nucleotides may involve mixing the various components together and exposing them to suitable amplification conditions for the low processivity polymerase being used. During amplification, primers attach to different portions of the single stranded genomic DNA template, and the low processivity polymerase introduces complementary free nucleotides into the template strand according to the sequence of the template strand. The low processivity polymerase naturally falls off of the template strand, usually after 100 bases or fewer have been replicated. The replicated complementary fragments can be displaced using, e.g., denaturation. The method may include repeating both the contacting and the displacing a predetermined number of cycles to generate additional complementary fragments in each of the predetermined number of cycles.

The following are some examples of this method.

The T4 DNA polymerase can be used for amplification of single stranded or denatured gDNA, for example, in 50 about mM N-(2-Hydroxyethyl)piperazine-N'-(2-ethanesulfonic acid) (HEPES) (pH 7.5), about 50 mM Tris-HCl (pH 8.6), or about 50 mM glycinate (pH 9.7). Example reaction mixtures may also include about 50 mM KCI, about 5 mM MgCl₂, about 5 mM dithiothreitol (DTT), about 40 µg/ml gDNA, about 0.2 mM of each dNTP, about 50 µg/ml bovine serum albumin (BSA), about 100 µM random primer (n=6) and about 10 units of T4 DNA polymerase incubated at 37°C for at least one hour. Temperature cycling may be used to displace replicate strands for multiple rounds of amplification.

Taq polymerase has low processivity at temperatures below 70°C. Accordingly, small fragments of gDNA can be obtained by using Taq polymerase at a low temperature, or in another condition in which Taq has low processivity. In another example, the Stoffel Fragment, which lacks the N-terminal 289 amino acid residues of Taq polymerase and has low processivity at 70° C., can be used to generate relatively small gDNA fragments. Taq or Stoffel Fragment can be used to amplify single stranded or denatured DNA templates, and temperature cycling can be used to displace replicate strands for multiple rounds of amplification.

The Klenow fragment can be used for isothermal amplification of a genome to produce small genomic DNA fragments, for example, in a low salt (I=0.085) reaction incubated at a temperature between about 5°C and 37°C. Example buffers and pH conditions that can be used to amplify gDNA with the Klenow fragment include, for example, about 50 mM Tris HCl (pH 7 .5), about 5 mM MgCl₂, about 50 mM NaCl, about 50 µg/ml bovine serum albumin (BSA), about 0.2 mM of each dNTP, about 2 µg of random primer (n=6), about 10 ng gDNA template, and about 5 units of Klenow fragment incubated at 37°C for about 16 hours. Similar reactions can be run where one or more reaction components is omitted or substituted. For example, the buffer can be replaced with about 50 mM phosphate (pH 7.4) or other pH values may be used in the range of about 7.0 to 7.8. In another example, conditions for amplification using the Klenow fragment can include, for example, about 10 ng gDNA template, about 2 mM dNTPs, about 10 mM MgCl₂, about 0.5 U/µl (microliter) polymerase, about 50 uM (micromolar) random primer (n=6) and isothermal incubation at 37°C for 16 hours.

Another example of the amplification method disclosed herein involves contacting a single stranded genomic DNA template with a polymerase, a plurality of primers, and a mixture of free nucleotides including natural nucleotides and dideoxythymidine triphosphate (ddTTP), thereby generating truncated complementary fragments of the single stranded genomic DNA template; and displacing the truncated complementary fragments from the single stranded genomic DNA template, thereby generating at least some of the respective samples.

In this example, any suitable polymerase may be used. The ddTTP acts as a truncating agent, and thus a high processivity polymerase may be used. Any of the polymerases set forth herein may be used in this example method, as long as the processing conditions can be altered so that the polymerase exhibits a higher processivity (e.g., can synthesize more than 100 bases per polymerization event). In an example, the high processivity polymerase is selected from the group consisting of T4 DNA polymerase, T7 DNA polymerase, Taq polymerase, Stoffel Fragment, Klenow Fragment, Bsu DNA polymerase, Bst DNA polymerase, and an engineered polymerase. In this example method, the term "engineered polymerase" is any synthetic polymerase that is designed to synthesize more than 100 bases per polymerization event. High processivity polymerases can produce fragments that are 10 kb (kilobase) to 20 kb in length. Other suitable high processivity polymerases include Φ29 polymerase.

In this example, any of the random primers described herein may be used.

In this example method, the free nucleotides in the mixture include natural nucleotides and dideoxythymidine triphosphate (ddTTP). The dideoxythymidine triphosphate serves as a synthesis terminating nucleotide or truncating agent. In an example, the natural nucleotides include deoxyadenine triphosphate, deoxythymine triphosphate, deoxyguanine triphosphate, and deoxycytosine triphosphate. In an example, the mixture of free nucleotides includes a ratio of deoxythymine triphosphate (dTTP) to dideoxythymidine triphosphate (ddTTP) ranging from about 10:5 to about 10:0.01. A ratio within this range helps to ensure that a desired number of deoxythymine triphosphate are incorporated into the generated fragments before the dideoxythymidine triphosphate truncates amplification. In one specific example, the ratio of dTTP to ddTTP is about 10:1.

Contacting the single stranded genomic DNA template with the polymerase, a plurality of primers, and the free nucleotide mixture may involve mixing the various components together and exosing them to suitable amplification conditions for the polymerase being used. During amplification, primers attach to different portions of the single stranded genomic DNA template, and the polymerase introduces complementary natural nucleotides into the template strand according to the sequence of the template strand. When dideoxythymidine triphosphate (ddTTP) is introduced instead of deoxythymidine triphosphate (dTTP), the amplification of the particular strand is terminated. As such, the ddTTP truncates the replicated DNA fragment. The ratio of dTTP to ddTTP in the mixture helps to ensure that the generated fragments are sufficienly long for subsequent analysis.

The replicated truncated complementary fragments can be displaced using, e.g., denaturation. The method may include repeating both the contacting and the displacing a predetermined number of cycles to generate additional truncated complementary fragments in each of the predetermined number of cycles.

Any of the buffers (e.g., HEPES, Tris-HCl, glycinate, etc.), salts (e.g., KCI, MgCl₂, etc.), redox reagents (e.g., dithiothreitol), and/or stabilizers (e.g., bovine serum albumin (BSA)) may be used in this example method in suitable amounts for high processivity.

In one specific example, T7 DNA polymerase has high processivity in the following reaction conditions: about 40 mM Tris-HCl, pH 7.5, about 15 mM MgCl₂, about 25 mM NaCl, about 5 mM DTT, about 0.25 mM of each dNTP, from about 0.00025 mM to about 0.125 mM of ddTTP, 50 µg/ml single stranded gDNA, about 100 µM random primer (n=6), from about 0.5 to about 1 unit of T7 DNA polymerase, reaction temperature greater than 37°C.

In another specific example, Taq polymerase is highly processive at temperatures around 70°C when reacted with a 10 fold molar excess of the DNA sample and the random primers (n=6). An amplification reaction run under these conditions can further include a buffer, such as Tris-HCl at about 20 mM, pH of about 7, from about 1 mM to 2 mM MgCl₂, about 0.2 mM of each dNTP, and from about 0.0002 mM to about 0.1 mM of ddTTP. Additionally, a stabilizing agent can be added, such as glycerol, gelatin, BSA, or a non-ionic detergent.

In yet another specific example, Klenow fragment has high processivity in the following reaction conditions: about 10 mM Tris-HCl, pH 7.9, about 10 mM MgCl₂, about 50 mM NaCl, about 1 mM DTT, and about 100 g/mol BSA.

In yet another specific example, Bst DNA polymerase has high processivity in the following reaction conditions: about 20 mM Tris-HCl, pH 8.8, about 10 mM (NH₄)₂SO₄, about 10 mM KCI, about 2 mM MgSO₄, and about 0.1% of a non-ionic surfactant (e.g., TRITON^{™} X-100 from The Dow Chemical Co.).

Both of the amplification processes disclosed herein generate a plurality of gDNA fragments without having to use a fragmentation process. The single stranded gDNA serves as a template for several target genotyping loci 54, and several amplicons of each target genotyping locus 54 are generated. When introduced onto a flow cell 20 with the templates 48 or 49 to be genotyped, the amplified gDNA fragments (target genotyping loci 54) hybridize to respective complementary sections of the templates 48 or 49 to be genotyped.

### Kits

The genotyping nucleotides 10 or 10' and the flow cell 20 may be part of a genotyping kit.

In one example, a kit comprises: a flow cell 20 including a substrate 30, 30' having depressions 38 separated by interstitial regions 40 and first and second capture primers 22, 24, or 22, 24' attached within each of the depressions 38; and a genotyping probe fluid including a liquid carrier and a genotyping oligonucleotide 10 or 10' in the liquid carrier, the genotyping oligonucleotide 10 or 10' including a first primer sequence 12, a probe sequence 14 representative of a target genotyping locus 54, a restriction endonuclease site 16, 16', and a second primer sequence 18, 18' that is at least partially complementary to the second capture primer 24, 24'.

The kit may also include genotyping library preparation components, such as a whole genome sample, a polymerase (which may be a low processivity polymerase as defined herein), a plurality of primers, and free nucleotides (in some examples including natural nucleotides, and in other examples including a mixture of natural nucleotides and dideoxythymidine triphosphate (ddTTP)).

Any example of the genotyping oligonucleotide 10 or 10' may be used in the kit and any example of the flow cell 20 may be used in the kit.

The kit may alternatively include a non-patterned flow cell with primers across the entire surface of a flow channel.

## Claims

1. A genotyping probe fluid, comprising:
a liquid carrier; and
a genotyping oligonucleotide in the liquid carrier, the genotyping oligonucleotide including:
a first primer sequence;
an index sequence portion directly attached to the first primer sequence;
a priming site portion directly attached to the index sequence portion;
a probe sequence directly attached to the priming site portion, the probe sequence being representative of a target genotyping locus having a nucleobase of interest, wherein a last base of the probe sequence is representative of a nucleobase of the target genotyping locus that is positioned directly adjacent to the nucleobase of interest;
a restriction endonuclease site; and
a second primer sequence that is at least partially complementary to a flow cell capture primer,
wherein the restriction endonuclease site is positioned between the probe sequence and the second primer sequence, or
the probe sequence is directly attached to the second primer sequence and the restriction endonuclease site is integrated into the second primer sequence at a cleavage distance from the last base of the probe sequence.

2. The genotyping probe fluid as defined in claim 1, further comprising a plurality of genotyping oligonucleotides in the liquid carrier, wherein each genotyping oligonucleotide includes a different probe sequence than each other genotyping oligonucleotide.

3. The genotyping probe fluid as defined in claim 1 or 2, wherein the restriction endonuclease site is sensitive to a restriction endonuclease selected from the group consisting of a 4 base cutter restriction endonuclease, a 5 base cutter restriction endonuclease, and a 6 base cutter restriction endonuclease.

4. A kit, comprising:
a flow cell, including:
a substrate; and
first and second capture primers attached to the substrate, the second capture primer having a restriction endonuclease site;
a genotyping probe fluid, including:
a liquid carrier; and
a genotyping oligonucleotide in the liquid carrier, the genotyping oligonucleotide including:
a first primer sequence;
an index sequence portion directly attached to the first primer sequence;
a priming site portion directly attached to the index sequence portion;
a probe sequence directly attached to the priming site portion, the probe sequence being representative of a target genotyping locus having a nucleobase of interest, wherein a last base of the probe sequence is representative of a nucleobase of the target genotyping locus that is positioned directly adjacent to the nucleobase of interest;
a second restriction endonuclease site that is complementary to the restriction endonuclease site of the second capture primer; and
a second primer sequence that is at least partially complementary to the second capture primer,
wherein the restriction endonuclease site is positioned between the probe sequence and the second primer sequence, or
the probe sequence is directly attached to the second primer sequence and the restriction endonuclease site is integrated into the second primer sequence at a cleavage distance from the last base of the probe sequence; and
genotyping library preparation components, including:
a polymerase;
a plurality of random primers; and
free nucleotides.

5. The kit as defined in claim 4, wherein:
the polymerase is a high processivity polymerase selected from the group consisting of T4 DNA polymerase, T7 DNA polymerase, Taq polymerase, Stoffel Fragment, Klenow Fragment, Bsu DNA polymerase, Bst DNA polymerase, and an engineered polymerase; and
the free nucleotides include a mixture of natural nucleotides and dideoxythymidine triphosphate (ddTTP).

6. The kit as defined in claim 4, wherein:
the polymerase is a low processivity polymerase selected from the group consisting of T4 DNA polymerase, T7 DNA polymerase, Taq polymerase, Stoffel Fragment, Klenow Fragment, Bsu DNA polymerase, Bst DNA polymerase, monomeric *E*. *coli* Pol III or *E*. *coli* Pol I, and an engineered polymerase; and
the free nucleotides include deoxyadenine triphosphate, deoxythymine triphosphate, deoxyguanine triphosphate, and deoxycytosine triphosphate.

7. The kit as defined in one of claims 4 through 6, wherein the plurality of random primers is a population of random primers synthesized to include a higher content of guanine and/or cytosine nucleotides compared to adenine and thymidine nucleotides.

8. The kit as defined in claim 4 or 7, wherein the restriction endonuclease site and the second restriction endonuclease site are sensitive to a Type IIS methyl sensitive restriction endonuclease.

9. A method, comprising:
introducing a genotyping probe fluid as defined in claim 4 to a flow cell as defined in claim 4, the genotyping probe fluid including a plurality of genotyping oligonucleotides as defined in claim 4; whereby a respective genotyping oligonucleotide reacts to produce respective clonal populations of amplicons from the respective genotyping oligonucleotide;
linearizing the amplicons to produce probe templates;
sequencing at least one probe identifying section of the probe templates to identify each of the probe sequences;
removing at least respective nascent strands from the probe templates, whereby a 3' OH group at an end of the probe templates is exposed;
hybridizing respective samples to the probe templates; and
performing respective genotyping reactions of the samples at the exposed 3' OH groups.

10. The method as defined in claim 9, wherein, prior to hybridizing the respective samples to the probe templates, the method further comprises preparing the respective samples by:
contacting a single stranded genomic DNA template with a polymerase, a plurality of primers, and a mixture of free nucleotides including natural nucleotides and dideoxythymidine triphosphate, thereby generating truncated complementary fragments of the single stranded genomic DNA template; and
displacing the truncated complementary fragments from the single stranded genomic DNA template.

11. The method as defined in claim 10, wherein the natural nucleotides include deoxyadenine triphosphate, deoxythymine triphosphate, deoxyguanine triphosphate, and deoxycytosine triphosphate, and wherein the mixture of free nucleotides includes a ratio of deoxythymine triphosphate to dideoxythymidine triphosphate ranging from about 10:5 to about 10:0.01.

12. The method as defined in claim 10 or 11, further comprising denaturing a double stranded genomic deoxyribonucleic acid (DNA), thereby generating the single stranded genomic DNA template.

13. The method as defined in one of claims 10 through 12, further comprising repeating both the contacting and the displacing a predetermined number of cycles to generate additional truncated complementary fragments in each of the predetermined number of cycles.

14. The method as defined in one of claims 10 through 13, wherein the polymerase is selected from the group consisting of T4 DNA polymerase, T7 DNA polymerase, Taq polymerase, Stoffel Fragment, Klenow Fragment, Bsu DNA polymerase, Bst DNA polymerase, and an engineered polymerase.

## Patentansprüche

1. Genotypisierungssondenfluid, umfassend:
einen flüssigen Träger; und
ein genotypisierendes Oligonukleotid in dem flüssigen Träger, wobei das genotypisierende Oligonukleotid einschließt:
eine erste Primersequenz;
einen Indexsequenzabschnitt, der direkt an der ersten Primersequenz anhaftet;
einen Primingstellenabschnitt, der direkt an dem Indexsequenzabschnitt anhaftet;
eine Sondensequenz, die direkt an dem Primingstellenabschnitt anhaftet, wobei die Sondensequenz repräsentativ für einen Ziel-Genotypisierungslocus, aufweisend eine Nukleobase von Interesse, ist, wobei eine letzte Base der Sondensequenz repräsentativ für eine Nukleobase des Ziel-Genotypisierungslocus ist, die direkt neben der Nukleobase von Interesse positioniert ist;
eine Restriktionsendonukleasestelle; und
eine zweite Primersequenz, die zumindest teilweise komplementär zu einem Durchflusszelleneinfangprimer ist,
wobei die Restriktionsendonukleasestelle zwischen der Sondensequenz und der zweiten Primersequenz positioniert ist, oder
die Sondensequenz direkt an der zweiten Primersequenz anhaftet und die Restriktionsendonukleasestelle in die zweite Primersequenz in einem Spaltungsabstand von der letzten Base der Sondensequenz integriert ist.

2. Genotypisierungssondenfluid wie in Anspruch 1 definiert, die ferner eine Vielzahl von Genotypisierungsoligonukleotiden in dem flüssigen Träger umfasst, wobei jedes Genotypisierungsoligonukleotid eine andere Sondensequenz als jedes andere Genotypisierungsoligonukleotid einschließt.

3. Genotypisierungssondenfluid wie in Anspruch 1 oder 2 definiert, wobei die Restriktionsendonukleasestelle für eine Restriktionsendonuklease empfindlich ist, die aus der Gruppe, bestehend aus einer 4-Basen-Cutter-Restriktionsendonuklease, einer 5-Basen-Cutter-Restriktionsendonuklease und einer 6-Basen-Cutter-Restriktionsendonuklease ausgewählt ist.

4. Kit, umfassend: eine Durchflusszelle, einschließend:
ein Substrat; und
erste und zweite Einfangprimer, die an dem Substrat anhaften, wobei der zweite Einfangprimer eine Restriktionsendonukleasestelle aufweist;
eine Genotypisierungssondenfluid, einschließend:
einen flüssigen Träger; und
ein genotypisierendes Oligonukleotid in dem flüssigen Träger, wobei das genotypisierende Oligonukleotid einschließt:
eine erste Primersequenz;
einen Indexsequenzabschnitt, der direkt an der ersten Primersequenz anhaftet;
einen Primingstellenabschnitt, der direkt an dem Indexsequenzabschnitt anhaftet;
eine Sondensequenz, die direkt an dem Primingstellenabschnitt anhaftet, wobei die Sondensequenz repräsentativ für einen Ziel-Genotypisierungslocus, aufweisend eine Nukleobase von Interesse, ist, wobei eine letzte Base der Sondensequenz repräsentativ für eine Nukleobase des Ziel-Genotypisierungslocus ist, die direkt neben der Nukleobase von Interesse positioniert ist;
eine zweite Restriktionsendonukleasestelle, die komplementär zu der Restriktionsendonukleasestelle des zweiten Einfangprimers ist; und
eine zweite Primersequenz, die zumindest teilweise komplementär zu dem zweiten Einfangprimer ist,
wobei die Restriktionsendonukleasestelle zwischen der Sondensequenz und der zweiten Primersequenz positioniert ist, oder
die Sondensequenz direkt an der zweiten Primersequenz anhaftet und die Restriktionsendonukleasestelle in die zweite Primersequenz in einem Spaltungsabstand von der letzten Base der Sondensequenz integriert ist; und Komponenten zur Herstellung einer Genotypisierungsbibliothek, einschließend:
eine Polymerase;
eine Vielzahl von Zufallsprimern und freien Nukleotiden.

5. Kit wie in Anspruch 4 definiert, wobei:
die Polymerase eine Polymerase mit hoher Verarbeitungskapazität ist, ausgewählt aus der Gruppe, bestehend aus T4-DNA-Polymerase, T7-DNA-Polymerase, Taq-Polymerase, Stoffel-Fragment, Klenow-Fragment, Bsu-DNA-Polymerase, Bst-DNA-Polymerase und einer veränderten Polymerase; und
die freien Nukleotide eine Mischung aus natürlichen Nukleotiden und Dideoxythymidintriphosphat (ddTTP) einschließen.

6. Kit wie in Anspruch 4 definiert, wobei:
die Polymerase eine Polymerase mit geringer Verarbeitungskapazität ist, ausgewählt aus der Gruppe, bestehend T4-DNA-Polymerase, T7-DNA-Polymerase, Taq-Polymerase, Stoffel-Fragment, Klenow-Fragment, Bsu-DNA-Polymerase, Bst-DNA-Polymerase, monomerem E. coli Pol III oder E. coli Pol I und einer veränderten Polymerase; und
die freien Nukleotide einschließen Desoxyadenintriphosphat, Desoxythymintriphosphat, Desoxyguanintriphosphat und Desoxycytosintriphosphat.

7. Kit wie in einem der Ansprüche 4 bis 6 definiert, wobei die Vielzahl von Zufallsprimern eine Population von Zufallsprimern ist, die so synthetisiert sind, dass sie einen höheren Gehalt an Guanin- und/oder Cytosin-Nukleotiden im Vergleich zu Adenin- und Thymidin-Nukleotiden einschließen.

8. Kit wie in Anspruch 1 oder 7 definiert, wobei die Restriktionsendonukleasestelle und die zweite Restriktionsendonukleasestelle empfindlich für eine methylsensitive Restriktionsendonuklease vom Typ IIS sind.

9. Verfahren, umfassend:
Einführen eines genotypisierenden Sondenfluids wie in Anspruch 4 definiert in eine Durchflusszelle wie in Anspruch 4 definiert, wobei das Genotypisierungssondenfluid eine Vielzahl von genotypisierenden Oligonukleotiden wie in Anspruch 4 definiert einschließt; wodurch ein jeweiliges genotypisierendes Oligonukleotid reagiert, um jeweilige klonale Populationen von Amplikonen aus dem jeweiligen genotypisierenden Oligonukleotid herzustellen;
Linearisierung der Amplikons zum Herstellen von Sonden-Templates;
Sequenzierung mindestens eines sondenidentifizierenden Abschnitts der Sonden-Templates, um jede der Sondensequenzen zu identifizieren;
Entfernen mindestens der jeweiligen naszierenden Stränge von den Sonden-Templates, wodurch eine 3'-OH-Gruppe an einem Ende der Sonden-Templates exponiert wird;
Hybridisierung der jeweiligen Proben mit den Sonden-Templates; und
Durchführung entsprechender Genotypisierungsreaktionen der Proben an den exponierten 3'-OH-Gruppen.

10. Verfahren wie in Anspruch 9 definiert, wobei das Verfahren vor der Hybridisierung der jeweiligen Proben mit den Sonden-Templates ferner das Herstellen der jeweiligen Proben umfasst durch:
Inkontaktbringen eines einzelsträngigen genomischen DNA-Templates mit einer Polymerase, einer Vielzahl von Primern und einem Gemisch freier Nukleotide einschließlich natürlicher Nukleotide und Dideoxythymidintriphosphat, wodurch trunkierte komplementäre Fragmente des einzelsträngigen genomischen DNA-Templates erzeugt werden; und
Verdrängen der trunkierten komplementären Fragmente aus dem einzelsträngigen genomischen DNA-Template.

11. Verfahren wie in Anspruch 10 definiert, wobei die natürlichen Nukleotide Desoxyadenintriphosphat, Desoxythymintriphosphat, Desoxyguanintriphosphat und Desoxycytosintriphosphat einschließen und wobei das Gemisch der freien Nukleotide ein Verhältnis von Desoxythymintriphosphat zu Didesoxythymidintriphosphat im Bereich von etwa 10:5 bis etwa 10:0,01 einschließt.

12. Verfahren wie in Anspruch 10 oder 11 definiert, ferner umfassend die Denaturierung einer doppelsträngigen genomischen Desoxyribonukleinsäure (DNA), wodurch das einzelsträngige genomische DNA-Template erzeugt wird.

13. Verfahren wie in einem der Ansprüche 10 bis 12 definiert, ferner umfassend das Wiederholen sowohl des Inkontaktbringens als auch des Verdrängens in einer vorbestimmten Anzahl von Zyklen, um in jedem der vorbestimmten Anzahl von Zyklen zusätzliche trunkierte komplementäre Fragmente zu erzeugen.

14. Verfahren wie in einem der Ansprüche 10 bis 13 definiert, wobei die Polymerase ausgewählt ist aus der Gruppe, bestehend aus T4-DNA-Polymerase, T7-DNA-Polymerase, Taq-Polymerase, Stoffel-Fragment, Klenow-Fragment, Bsu-DNA-Polymerase, Bst-DNA-Polymerase und einer veränderten Polymerase.

## Revendications

1. Fluide sonde de génotypage, comprenant
une substance liquide de support ; et
un oligonucléotide de génotypage dans la substance liquide de support, l'oligonucléotide de génotypage incluant :
une première séquence d'amorce ;
une partie séquence index raccordée directement à la première séquence d'amorce ;
une partie site d'amorçage raccordée directement à la partie séquence index ;
une séquence de sonde raccordée directement à la partie site d'amorçage, la séquence de sonde étant représentative d'un locus de génotypage cible comportant une base nucléique d'intérêt, une dernière base de la séquence de sonde étant représentative d'une base nucléique du locus de génotypage cible qui est positionnée en étant directement adjacente à la base nucléique d'intérêt ;
un site pour endonucléase de restriction ; et
une seconde séquence d'amorce qui est au moins partiellement complémentaire d'une amorce de capture de cellule à flux continu,
dans lequel le site pour endonucléase de restriction est positionné entre la séquence de sonde et la seconde séquence d'amorce, ou
la séquence de sonde est raccordée directement à la seconde séquence d'amorce et le site pour endonucléase de restriction est intégré dans la seconde séquence d'amorce à une distance de clivage à partir de la dernière base de la séquence de sonde.

2. Fluide sonde de génotypage tel que défini dans la revendication 1, comprenant en outre une pluralité d'oligonucléotides de génotypage dans la substance liquide de support, chaque oligonucléotide de génotypage incluant une séquence de sonde différente de chaque autre oligonucléotide de génotypage.

3. Fluide sonde de génotypage tel que défini dans la revendication 1 ou 2, dans lequel le site pour endonucléase de restriction est sensible à une endonucléase de restriction choisie dans le groupe constitué par une endonucléase de restriction coupant au niveau de 4 bases, une endonucléase de restriction coupant au niveau de 5 bases, et une endonucléase de restriction coupant au niveau de 6 bases,

4. Kit, comprenant :
une cellule à flux continu, incluant
un substrat ; et
des première et seconde amorces de capture fixées au substrat, la seconde amorce de capture comportant un site pour endonucléase de restriction ;
un fluide sonde de génotypage, incluant :
une substance liquide de support ; et
un oligonucléotide de génotypage dans la substance liquide de support, l'oligonucléotide de génotypage incluant :
une première séquence d'amorce ;
une partie séquence index raccordée directement à la première séquence d'amorce ;
une partie site d'amorçage raccordée directement à la partie séquence index ;
une séquence de sonde raccordée directement à la partie site d'amorçage, la séquence de sonde étant représentative d'un locus de génotypage cible comportant une base nucléique d'intérêt, une dernière base de la séquence de sonde étant représentative d'une base nucléique du locus de génotypage cible qui est positionnée en étant directement adjacente à la base nucléique d'intérêt ;
un second site pour endonucléase de restriction qui est complémentaire du site pour endonucléase de restriction de la seconde amorce de capture ; et
une seconde séquence d'amorce qui est au moins partiellement complémentaire de la seconde amorce de capture,
le site pour endonucléase de restriction étant positionné entre la séquence de sonde et la seconde séquence d'amorce, ou
la séquence de sonde étant raccordée directement à la seconde séquence d'amorce et le site pour endonucléase de restriction étant intégré dans la seconde séquence d'amorce à une distance de clivage à partir de la dernière base de la séquence de sonde ; et
des composants de préparation de banque de génotypage, incluant :
une polymérase ;
une pluralité d'amorces aléatoires ; et
des nucléotides libres.

5. Kit tel que défini dans la revendication 4, dans lequel :
la polymérase est une polymérase à haute processivité, choisie dans le groupe constitué par l'ADN polymérase de T4, l'ADN polymérase de T7, la polymérase Taq, le fragment de Stoffel, le fragment de Klenow, l'ADN polymérase de Bsu, l'ADN polymérase de Bst, et une polymérase modifiée ; et
les nucléotides libres incluent un mélange de nucléotides naturels et de didésoxythymidine triphosphate (ddTTP).

6. Kit tel que défini dans la revendication 4, dans lequel :
la polymérase est une polymérase à faible processivité, choisie dans le groupe constitué par l'ADN polymérase de T4, l'ADN polymérase de T7, la polymérase Taq, le fragment de Stoffel, le fragment de Klenow, l'ADN polymérase de Bsu, l'ADN polymérase de Bst, Pol I de *E. coli* ou Pol III de *E. coli* monomère, et une polymérase modifiée ; et
les nucléotides libres incluent la désoxyadénine triphosphate, la désoxythymidine triphosphate, la désoxyguanine triphosphate, et la désoxycytosine triphosphate.

7. Kit tel que défini dans l'une quelconque des revendications 4 à 6, dans lequel la pluralité d'amorces aléatoires est une population d'amorces aléatoires synthétisées pour inclure une plus forte teneur en nucléotides à guanine et/ou cytosine en comparaison des nucléotides à adénine et thymidine.

8. Kit tel que défini dans la revendication 4 ou 7, dans lequel le site pour endonucléase de restriction et le second site pour endonucléase de restriction sont sensibles à une endonucléase de restriction de type IIS sensible à la méthylation.

9. Procédé, comprenant les étapes consistant à :
introduire un fluide sonde de génotypage tel que défini dans la revendication 4 dans une cellule à flux continu telle que définie dans la revendication 4, le fluide sonde de génotypage incluant une pluralité d'oligonucléotides de génotypage tels que définis dans la revendication 4 ; ce par quoi un oligonucléotide de génotypage respectif réagit pour produire des populations clonales respectives d'amplicons à partir de l'oligonucléotide de génotypage respectif ;
linéariser les amplicons pour produire des matrices de sonde ;
séquencer au moins un segment d'identification de sonde des matrices de sonde pour identifier chacune des séquences de sonde ;
éliminer au moins des brins naissants respectifs à partir des matrices de sonde, ce par quoi un groupe OH en 3' à une extrémité des matrices de sonde est exposé ;
hybrider des échantillons respectifs avec les matrices de sonde ; et
effectuer des réactions de génotypage respectives des échantillons au niveau des groupes OH en 3' exposés.

10. Procédé tel que défini dans la revendication 9, dans lequel, avant d'hybrider les échantillons respectifs avec les matrices de sonde, le procédé comprend en outre préparer les échantillons respectifs par :
mise en contact d'une matrice d'ADN génomique simple brin avec une polymérase, une pluralité d'amorces et un mélange de nucléotides libres incluant des nucléotides naturels et de la didésoxythymidine triphosphate, en engendrant ainsi des fragments complémentaires tronqués de la matrice d'ADN génomique simple brin ; et
déplacement des fragments complémentaires tronqués à partir de la matrice d'ADN génomique simple brin.

11. Procédé tel que défini dans la revendication 10, dans lequel les nucléotides naturels incluent la désoxyadénine triphosphate, la désoxythymidine triphosphate, la désoxyguanine triphosphate et la désoxycytosine triphosphate, et dans lequel le mélange des nucléotides libres inclut un rapport de désoxythymidine triphosphate à didésoxythymidine triphosphate se situant dans la plage d'environ 10:5 à environ 10:0,01.

12. Procédé tel que défini dans la revendication 10 ou 11, comprenant en outre dénaturer un acide désoxyribonucléique (ADN) génomique double brin, en engendrant ainsi la matrice d'ADN génomique simple brin.

13. Procédé tel que défini dans l'une quelconque des revendications 10 à 12, comprenant en outre répéter pendant un nombre préétabli de cycles à la fois la mise en contact et le déplacement pour engendrer des fragments complémentaires tronqués supplémentaires dans chacun du nombre préétabli de cycles.

14. Procédé tel que défini dans l'une quelconque des revendications 10 à 13, dans lequel la polymérase est choisie dans le groupe constitué par l'ADN polymérase de T4, l'ADN polymérase de T7, la polymérase Taq, le fragment de Stoffel, le fragment de Klenow, l'ADN polymérase de Bsu, l'ADN polymérase de Bst, et une polymérase modifiée.
